# EUROPEAN PATENT APPLICATION

(11) **EP 3 182 203 A1**
(43) Date of publication of application: **21.06.2017**
(21) Application number: 15201408.0
(22) Date of filing: 18.12.2015
(51) Int. Cl.: G03F 7/004, C07D 221/14

(54) **A COMBINATION OF NIT DERIVATIVES WITH SENSITIZERS**

(71) Applicant: Heraeus Precious Metals North America Daychem LLC, 45377 Vandalia, OH (US)
(72) Inventor: Lee, Yeong Beom, Ansan-City (KR); Cho, Hyun Yong, Suwon (KR); Zhang, Yongqiang, Centerville, OH Ohio 45458 (US); Kunz, Martin, 76337 Waldbronn (DE)
(74) Representative: Herzog, Fiesser & Partner Patentanwälte PartG mbB

(57) **Abstract**

The present invention relates to a composition comprising
(i) at least one sensitizer compound that, upon exposure to electromagnetic radiation, changes from a ground state into an excited state;
and
(ii) at least one acid generating compound that, upon transfer of energy or an electron from the sensitizer compound in its excited state to the acid generating compound, releases an acid, the acid generating compound having the general structure (I) 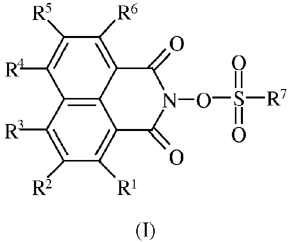 wherein
- R¹, R², R³, R⁴, R⁵ and R⁶ independently from each other represent a hydrogen atom or an organic residue, with the provisio that at least one of R¹, R², R³, R⁴, R⁵ and R⁶, preferably at least one of R² and R³, is an organic residue;
- R⁷ represents an organic residue.

The present invention also relates to a process for producing a composite, to the composite obtained by the process according to the present invention, to a composite comprising a substrate and a coating applied on the surface of the substrate in a patterned structure and to the use of a combination of a sensitizer compound and an acid generating compound for photoinduced polymerization, photoinduced crosslinking, photoinduced degradation and photoinduced transformation of functional groups.

## Description

The present invention relates to a composition comprising at least one sensitizer compound that, upon exposure to electromagnetic radiation, changes from a ground state into an excited state, and at least one acid generating compound that, upon transfer of energy or an electron from the sensitizer compound in its excited state to the acid generating compound, releases an acid, to a process for producing a composite, to the composite obtained by the process according to the present invention, to a composite comprising a substrate and a coating applied on the surface of the substrate in a patterned structure and to the use of a combination of a sensitizer compound and an acid generating compound for photoinduced polymerization, photoinduced crosslinking, photoinduced degradation and photoinduced transformation of functional groups.

Photoresists are photosensitive films for transfer of images to a substrate. They form negative or positive images. After coating a photoresist on a substrate, the coating is exposed through a patterned photomask to a source of activating energy, such as ultraviolet light, to form a latent image in the photoresist coating. The photomask has areas opaque and transparent to activating radiation that define an image desired to be transferred to the underlying substrate.

Chemical amplification-type photoresists have proven to be useful in achieving high sensitivity in processes for forming ultrafine patterns in the manufacture of semiconductors. These photoresists are prepared by blending a photoacid generator ("PAG") with a polymer matrix having acid labile structures. According to the reaction mechanism of such a photoresist, the photoacid generator generates acid when it is irradiated by the light source, and the main chain or branched chain of the polymer matrix in the exposed or irradiated portion reacts in a so called *"post exposure bake"* (PEB) with the generated acid and is decomposed or cross-linked, so that the polarity of the polymer is considerably altered. This alteration of polarity results in a solubility difference in the developing solution between the irradiated exposed area and the unexposed area, thereby forming a positive or negative image of a mask on the substrate. Acid diffusion is important not only to increase photoresist sensitivity and throughput, but also to limit line edge roughness due to shot noise statistics.

In a chemically amplified photoresist, the solubility-switching chemistry necessary for imaging is not caused directly by the exposure; rather exposure generates a stable catalytic species that promotes solubility-switching chemical reactions during the subsequent PEB step. The term "chemical amplification" arises from the fact that each photochemically-generated catalyst molecule can promote many solubility-switching reaction events. The apparent quantum efficiency of the switching reaction is the quantum efficiency of catalyst generation multiplied by the average catalytic chain length. The original exposure dose is "amplified" by the subsequent chain of chemical reaction events. The catalytic chain length for a catalyst can be very long (up to several hundred reaction events) giving dramatic exposure amplification.

Chemical amplification is advantageous in that it can greatly improve resist sensitivity, but it is not without potential drawbacks. For instance as a catalyst molecule moves around to the several hundred reactions sites, nothing necessarily limits it to the region that was exposed to the imaging radiation. There is a potential trade-off between resist sensitivity and imaging fidelity. For example, the amplified photoresist is exposed through a photomask, generating acid catalyst in the exposed regions. The latent acid image generated in the first step is converted into an image of soluble and insoluble regions by raising the temperature of the wafer in the PEB, which allows chemical reactions to occur. Some acid migrates out of the originally exposed region causing "critical dimension bias" problems. After baking, the image is developed with a solvent. The developed feature width may be larger than the nominal mask dimension as the result of acid diffusion from exposed into the unexposed regions. For much of the history of amplified resists this trade-off was of little concern as the catalyst diffusion distances were insignificant relative to the printed feature size, but as feature sizes have decreased, the diffusion distances have remained roughly the same and catalyst diffusion has emerged as a significant concern.

In order to generate enough acid which would change the solubility of the polymer, a certain exposure time is required. For a known PAG molecule like N-hydroxynaphthalimide triflate ("NIT"), this exposure time is rather long (due to its low absorption at 365 nm or longer). Increasing the concentration of such PAGs, however, will not result in faster exposure times because the solubility of the PAG is the limiting factor. Another possibility is to add sensitizers which absorb the light and transfer energy to the PAG which would then liberate the acid. Such sensitizers, however, must be used in rather high concentrations in order to be able to transfer the energy to a PAG in close proximity. At such high concentrations, sensitizers often have an absorption which is too high and has negative effects on the shape of the resist profile after development.

Higher sensitivity is also needed in any application where effects based on a photo generation of acid is used. In addition to resist applications these applications could be for photoinduced polymerization, photoinduced crosslinking, photoinduced degradation, photoinduced deprotection, photoinduced color change or photoinduced transformation of functional groups or any combination of at least two of them

It was therefore an object of the present invention to overcome the disadvantages of the prior art in the field of chemical amplification-type photoresists.

In particular, it was an object of the present invention to provide photoresist compositions having has a high sensitivity towards electromagnetic radiation, in particular towards electromagnetic radiation with a wavelength in the range of 200 to 500 nm, more particularly towards electromagnetic radiation with a wavelength of 365 nm (i-line), and which preferably also allows the production of a patterned structure with a higher resolution, compared to the photoresist compositions known from the prior art.

### EMBODIMENTS

|1| A composition comprising
   (i) at least one sensitizer compound that, upon exposure to electromagnetic radiation, changes from a ground state into an excited state; and
   (ii) at least one acid generating compound that, upon transfer of energy or an electron from the sensitizer compound in its excited state to the acid generating compound, releases an acid, the acid generating compound having the general structure (I) 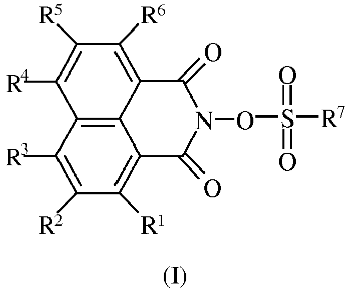 wherein
      - R¹, R², R³, R⁴, R⁵ and R⁶ independently from each other represent a hydrogen atom or an organic residue, with the provisio that at least one of R¹, R², R³, R⁴, R⁵ and R⁶, preferably at least one of R² and R³, is an organic residue;
      - R⁷ represents an organic residue.
|2| The composition according to embodiment |1|, wherein the sensitizer compound (i) is selected from the group consisting of xanthones, thioxanthones, coumarines, anthracenes, acridines, imidazoles, triazines, amino benzoates and a mixture of at least two thereof.
|3| The composition according to embodiment |1| or|2|, wherein the sensitizer compound
   (i) has the general structure (II) 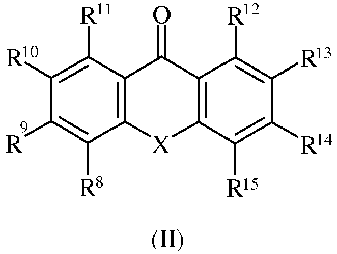 wherein R⁸, R⁹, R¹⁰, R¹¹, R¹², R¹³, R¹⁴ and R¹⁵ independently from each other represent a hydrogen atom, an organic residue, a hydroxy group, an amine group, a nitro group or a halogen, wherein two of these residues may form together an aliphatic or aromatic ring system, and X represents either O or S.
|4| The composition according to embodiment |3|, wherein R⁸, R⁹, R¹⁰, R¹¹, R¹², R¹³, R¹⁴ and R¹⁵ are selected from the group consisting of H, Cl, Br, I, -OH, -NO₂, -CN, -CH₃, -CH₂CH₃, -CH(CH)₃, -C(CH₃)₃, -O-CH₃, -O-CH₂CH₃, -O-CH₂CH₂CH₃, -C(=O)-O-CH₃, -C(=O)-O-CH₂CH₃, -C(=O)CH₃, -C(=O)CH₂CH₃, -C(=O)CH₂CH₂CH₃, -NH₂, -NHCH₃, -N(CH₃)₂, -N(CH₃)₂, -NH(CH₂CH₃), -N(CH₂CH₃)₂, -NH(CH₂CH₂OH), -N(CH₂CH₂OH)₂, -SO₂Ph and -O-CH₂-CH(OH)-CH₂-N⁺(CH₃)₃Cl⁻.
|5| The composition according to embodiment |3|, wherein the sensitizer compound is selected from the group consisting of thioxanthone, 2-isopropylthioxanthone, 4-isopropyl-thioxanthone, 2,4-diethylthioxanthone, (2-hydroxy-3-(3,4-dimethyl-9-oxo-9H-thio-xanthen-2-yloxy)-N,N,N-trimethyl-1-propanium chloride, 1-chloro-4-propoxythio-xanthone, 2-chlorothioxanthone, thioxanthone-anthracene and mixtures of at least two thereof.
|6| The composition according to anyone of embodiments |1| to |5|, wherein the solubility of the acid generating compound (ii) in propylene glycol monomethyl ether acetate (PGMEA) is at least 2 wt.-%.
|7| The composition according to anyone of embodiments |1| to |6|, wherein the acid generating compound (ii) has the general structure (Ia) or (Ib) 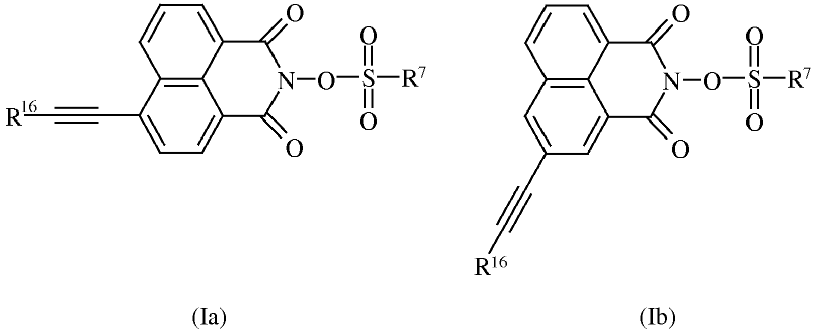 wherein
   R¹⁶ is selected from the group consisting of
      - a hydrogen atom,
      - an aliphatic group having a carbon number of from 1 to 18 in which one or more hydrogen atoms may be substituted by a halogen atom,
         and
      - an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
   R⁷ is selected from the group consisting of
      - -CH₃, -CH₂F, -CHF₂, or-CF₃,
      - an aliphatic group having a carbon number of from 2 to 18, which may be substituted by one or more halogen atom(s),
      - an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
      - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
         and
      - an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.
|8| The composition according to embodiment |7|, wherein R⁷ is -CF₃ and R¹⁶ is selected from the group consisting of
   - a hydrogen atom,
   - an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NRa-, and -C(=O)-NR^{a}R^{b}, wherein R^{a} and R^{b} are as defined above, wherein the aliphatic group optionally comprises at least one halogen atom,
   - -CH₂CH(CH₃)₂, -CH₂CH=CHCH₃, or -CH₂CH₂CH=CH₂,
   - a group represented by the Formula (A):

      -CH₂-R¹⁷ (A)

      wherein R¹⁷ is selected from the group consisiting of an aliphatic group having a carbon number of from 4 to 18,
   - cyclopropenyl,
      and
   - a group represented by the Formula (B): 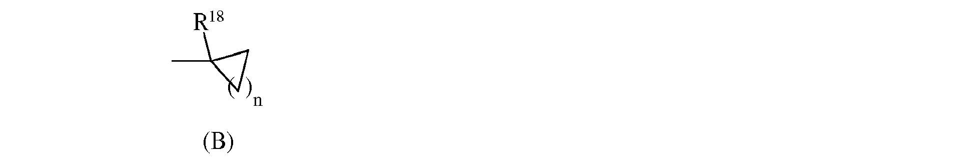 wherein R¹⁸ is selected from the group consisting of a hydrogen atom or an alkyl group having a carbon number of from 1 to 10; and n is equal to 1 to 5.
|9| The composition according to embodiment |7| or |8|, wherein the acid generating compound (ii) is selected from the group consisting of compounds T1 to T41 as described below.
|10| The composition according to anyone of embodiments |1| to |6|, wherein the acid generating compound (ii) has the general structure (Ic) or (Id) 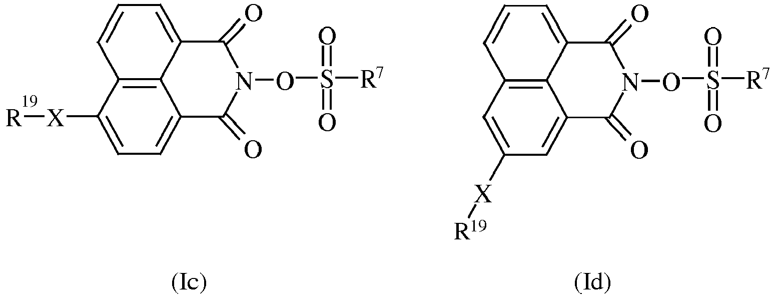 wherein
   X is O or S;
   R¹⁹ is selected from the group consisting of
      - an aliphatic group having a carbon number of from 1 to 18 in which one or more hydrogen atoms may be substituted by a halogen atom,
      - an aliphatic group having a carbon number of from 2 to 18 which comprises at least one moiety selected from the group consisting of -S-, -C(=O)-S-, -O-S(=O)₂-, -O-C(=O)-O-, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
      - a group represented by the Formula (C):

         -R²⁰-Ar (C)

         wherein
         - R²⁰ is a single bond or an aliphatic group having a carbon number of from 1 to 20, which may comprise at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
            and
         - Ar is an aryl or heteroaryl group in which one or more hydrogen atoms may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
      - a group represented by the Formula (D):

         -R²¹-R²²-O-C(=O)-R²³-R²³ (D)

         wherein
         - R²¹ and R²² are each independently a aliphatic group having a carbon number of from 1 to 5,
         - R²³ is an aliphatic group having a carbon number of from 1 to 10; and
         - R²⁴ is an aliphatic group having a carbon number of from 1 to 18, which comprises at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, and wherein the at least one moiety, if more than one, may be separated by an aliphatic group,
         and
      - a group represented by the Formula (E):

         -R²⁵-C(=O)-O-R²⁶ (E)

         wherein
         - R²⁵ is an aliphatic group having a carbon number of from 2 to 18, which may comprise at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
            and
         - R²⁶ is an aliphatic group having a carbon number of from 1 to 18 which comprises at least one moiety selected from the group consisting of -O-, -S-, -C(=O)-O-,-C(=O)-S-, -O-S(=O)₂-, -O-C(=O)-O-, -C(=O)-NH-, -O-C(=O)-NH-, -C(=O)-NRₐ-, -O-C(=O)-NR^{a}-, and -C(=O)-NR^{a}R^{b}, wherein R^{a} and R^{b} are as defined above, and wherein the at least one moiety, if more than one, may be separated by a aliphatic group,
   R⁷ is selected from the group consisting of
      - an aliphatic group having a carbon number of from 1 to 18, which may be substituted by one or more halogen atom(s),
      - an aliphatic group having a carbon number of from 3 to 18 which comprises at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
      - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
         and
      - an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.
|11| The composition according to embodiment |10|, wherein the acid generating compound (ii) is selected from the group consisting of compounds S-1, S-2, S-3, S-4, O-29, O-30, O-45, O-46, O-47, O-48, O-49, O-50, O-51, O-52, S-15, S-17, S-18, S-22, S-23, S-24, S-11, S-16, S-34, S-35, S-36, S-37, S-5, S-6, S-7, S-8, S-9, O-12, S-13, S-14, S-19, S-20, S-21, S-39, S-40, O-41, O-42, O-44, S-10, S-43, S-25 and S-33 as described below.
|12| The composition according to anyone of embodiments |1| to |6|, wherein the acid generating compound (ii) has a general structure (I) in which at least one of R¹, R², R³, R⁴, R⁵ and R⁶ represents a group of the Formula (Fa) or (Fb)

   -NR²⁷R²⁸ (Fa)

   -A-NR²⁷R²⁸ (Fb)

   wherein
   - R²⁷ and R²⁸: independently from each other represent a hydrogen atom, a linear or branched, optionally substituted aliphatic or heteroaliphatic group having 1-20 carbon atoms or an optionally substituted aromatic or heteroaromatic group having 6-20 carbon atoms, or
   - R²⁷ and R²⁸: together form a heterocyclic group that, in addition to the nitrogen atom shown in Formula (Fa) or (Fb), optionally comprises a further heteroatom selected from the group consisiting of N, O and S;
   - A: represents an optionally substituted divalent aliphatic or heteroaliphatic group having 1-20 carbon atoms or a divalent aromatic or heteroaromatic group having 6-20 carbon atoms.
|13| The composition according to embodiment |12|, wherein R⁷ is a linear, branched or cyclic perfluoroalkyl group having the general Formula -CₓF_{y}, in which x is an integer in the range from 1-8 and y is an integer in the range from 3-17.
|14| The composition according to embodiment |13|, wherein R⁷ is selected from the group consisting of -C₆F₅, -C₇F₇, -CF₃, -C₂F₅, -C₃F₇ and -C₄F₉.
|15| The composition according to anyone of embodiments |12| to |14|, wherein the acid generating compound (ii) has the general structure (Ie), (If), (Ig) or (Ih) 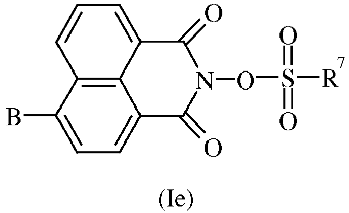 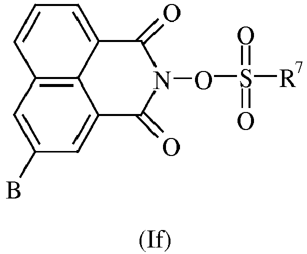 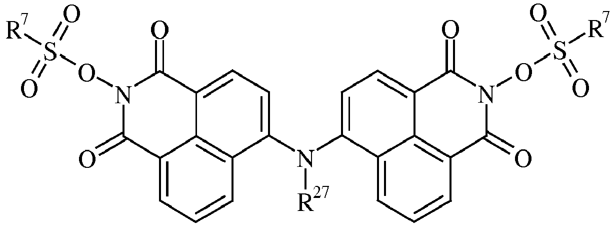 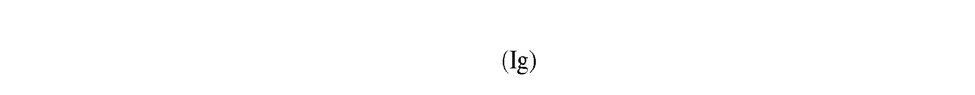 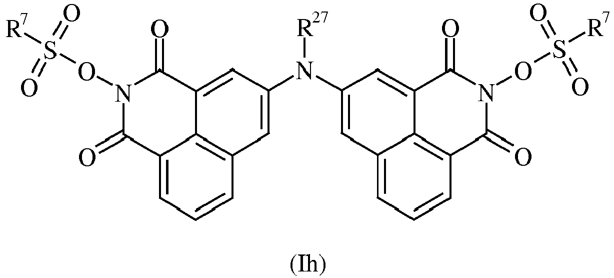 wherein B represents a group of the Formula (Fa) or (Fb) as defined in embodiment |12|, R²⁷ is as defined in embodiment |12| and R⁷ is as defined in embodiments |13| and |14|.
|16| The composition according to anyone of embodiments |12| to |15|, wherein the acid generating compound (ii) is selected from the group consisting of compounds Va to Vt as described below.
|17| The composition according to anyone of embodiments |1| to |6|, wherein the acid generating compound (ii) has the general structure (Ii) or (Ij) 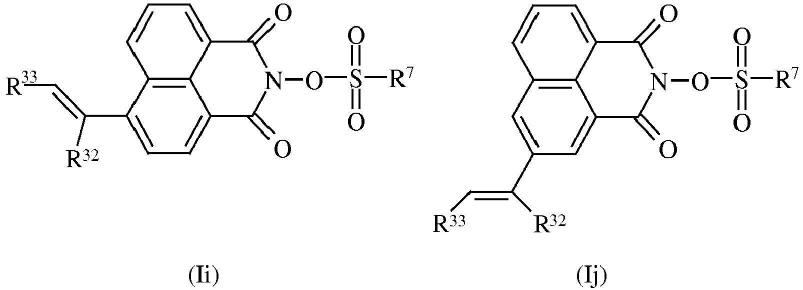 wherein
   R³² and R³³ may be the same or different, may be connected to from an alicylic or heterocyclic group and are independently selected from the group consisting of
      - a hydrogen atom,
      - a cyano group,
      - an aliphatic, alicyclic or heterocyclic group having a carbon number of from 1 to 18 in which one or more hydrogen atoms may be substituted by a halogen atom,
      - an aliphatic group having a carbon number of from 1 to 18 which comprises at least one moiety selected from the group consisting of -O-, -S-, -C(=O) -, -C(=O)-O-, -C(=O)-S-, -O-C(=O)-O-, -CN, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic or heterocyclic group, and wherein the aliphatic group optionally comprises at least one halogen atom,
         and
      - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
   R⁷ is selected from the group consisting of
      - an aliphatic, alicyclic or heterocyclic group having a carbon number of from 1 to 18, which may be substituted by one or more halogen atom(s),
      - an aliphatic or alicyclic group having a carbon number of from 1 to 18 which comprises at least one moiety selected from the group consisting of -O-, -S-, -C(=O), -C(=O)-O-, -C(=O)-S-, -O-C(=O)-O-, -CN, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
         and
      - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.
|18| The composition according to embodiment |17|, wherein the acid generating compound (ii) is selected from the group consisting of compounds D-1 to D-23 as described below.
|19| The composition according to anyone of embodiments |1| to |18|, wherein the molar ratio of the sensitizer compound (i) to the acid generating compound (ii) in the composition is in the range from 0.01 : 1 to 100 : 1, more preferably in the range from 0.25 : 1 to 10 : 1, even more preferably in the range from 0.05 : 1 to 5 : 1 and most preferably in the range from 0.1 : 1 to 1 : 1.
|20| The composition according to anyone of embodiments |1| to |19|, wherein the composition further comprises
   (iii) at least one compound capable of being imparted with an altered solubility in an aqueous solution in the presence of an acid;
   (iv) at least one organic solvent; and
   (v) optionally at least one additive being different from components (i), (ii), (iii) and (iv).
|21| The composition according to embodiment |20|, wherein the at least one compound (iii) is a poly(hydroxystyrene)-polymer in which at least a part of the hydroxy groups is substituted by protective groups.
|22| The composition according to embodiment |21|, wherein the protective group is selected from the group consisting of a tert-butoxycarbonyloxy group, a tert-butyloxy group, a tert-amyloxycarbonyloxy group and an acetal group.
|23| The composition according to anyone of embodiments |20| to |22|, wherein the at least one organic solvent (iv) is selected from the group consisting of an alcohol, a ketone, an ether, ester and a mixture of at least two thereof.
|24| The composition according to embodiment |23|, wherein the organic solvent (iv) is PGMEA.
|25| The composition according to anyone of embodiments |20| to |24|, wherein the composition comprises:
   (i) 0.01 to 40 wt.-%, preferably 0.01 to 10.0 wt.-% and more preferably 0.02 to 5.0 wt.-% of the sensitizer compound;
   (ii) 0.01 to 10 wt.-%, preferably 0.01 to 5.0 wt.-% and more preferably 0.02 to 4.0 wt.-% of the acid generating compound;
   (iii) 5 to 90 wt.-%, preferably 5 to 30 wt.-% and more preferably 5 to 20 wt.-% of the compound capable of being imparted with an altered solubility in an aqueous solution in the presence of an acid component;
   (v) 0 to 10 wt.-%, preferably 0.001 to 1.0 wt.-% and more preferably 0.01 to 0.1 wt.-% of the further additive;
   wherein the reminder in the composition is the organic solvent (iv).
|26| A process for producing a composite comprising a substrate and a coating that is applied onto the substrate in a patterned structure, the process comprising the steps of
   (α1) applying a layer of the composition according to anyone of embodiments |20| to |25| onto the surface of the substrate and at least partial removal of the organic solvent (iv);
   (α2) exposing selected areas of the layer to electromagnetic radiation, thereby releasing an acid from the acid generating compound (ii) in the areas exposed to the electromagnetic radiation;
   (α3) optionally heating the layer to impart compound (iii) in the areas in which the acid has been released with an altered solubility in an aqueous solution;
   (α4) optionally at least partial removal of the layer.
|27| A composite obtained by the process according to embodiment |26|.
|28| A composite comprising a substrate and a coating applied on the surface of the substrate in a patterned or non-patterned structure, wherein the coating comprises
   - a sensitizer compound (i) as defined in embodiments |1| to |5|
      and
   - an acid generating compound (ii) as defined in embodiments |1| and |6| to |18| or the fragment thereof that remains if in general structure (I) the acid group is cleaved.
|29| The use of a combination of a sensitizer compound (i) as defined in embodiments |1| to |5| and an acid generating compound (ii) as defined in embodiments |1| and |6| to |18| for photoinduced polymerization, photoinduced crosslinking, photoinduced degradation, photoinduced deprotection, photoinduced color change, photoinduced transformation of functional groups or any combination of at least two of them.
|30| The use of a combination of a sensitizer compound (i) as defined in embodiments |1| to |5| and an acid generating compound (ii) as defined in embodiments |1| and |6| to |18| in protective coatings, printing plates, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufracturing.

A contribution towards solving these objects is made by a composition comprising
(i) at least one sensitizer compound that, upon exposure to electromagnetic radiation, changes from a ground state into an excited state;
   and
(ii) at least one acid generating compound that, upon transfer of energy or an electron from the sensitizer compound in its excited state to the acid generating compound, releases an acid, the acid generating compound having the general structure (I) 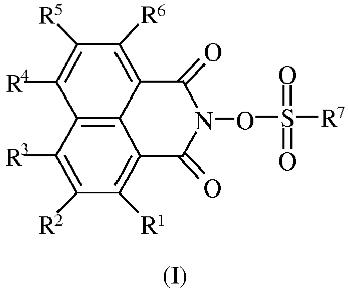
wherein
- R¹, R², R³, R⁴, R⁵ and R⁶ independently from each other represent a hydrogen atom or an organic residue, with the provisio that at least one of R¹, R², R³, R⁴, R⁵ and R⁶, preferably at least one of R² and R³, is an organic residue;
- R⁷ represents an organic residue.

Surprisingly, it has been discovered that using NIT derivatives which are substituted by organic residues at the aromatic ring system the solubility in organic solvents can be increased to such an extent that these derivatives can be used in combination with sensitizers to obtain photoresist compositions having has a high sensitivity towards electromagnetic radiation, in particular towards electromagnetic radiation with a wavelength in the range of 200 to 500 nm, more particularly towards electromagnetic radiation with a wavelength of 365 nm (i-line). In combination with these NIT derivatives the sensitizers can be used in concentrations which are effective enough to increase the amount of acid that is released by the derivative of NIT upon exposure of electromagnetic radiation, but which are not too high to negatively affect the shape of the resist profile after development. Using NIT derivatives which are substituted by an organic residue, a hydroxy group, an amine group, a nitro group and/or a halogen at the aromatic ring system in combination with sensitizers therefore allows the production of photoresist composition which are not only characterized by an increased sensitivity towards electromagnetic radiation, but also allow the production of a pattern with a high resolution.

In the composition according to the present invention the molar ratio of the sensitizer compound (i) to the acid generating compound (ii) is preferably in the range from 0.01 : 1 to 100 : 1, more preferably in the range from 0.25 : 1 to 10 : 1, even more preferably in the range from 0.05 : 1 to 5 : 1 and most preferably in the range from 0.1 : 1 to 1 : 1. If, for example, the composition comprises 0.5 mol sensitizer compound (i) and 1.0 mol acid generating compound (ii), the molar ratio of the sensitizer compound (i) to the acid generating compound (ii) is 0.5 (0.5/1.0).

### DEFINITIONS

In reference to the present disclosure, the technical and scientific terms used in the descriptions herein will have the meanings commonly understood by one of ordinary skill in the art, unless specifically defined otherwise. Accordingly, the following terms are intended to have the following meanings.

As used herein, the terms "residue" or "moiety" refer to a specific segment or functional group of a molecule. Chemical moieties are often recognized chemical entities embedded in or appended to a molecule.

As used herein the term "aliphatic" encompasses the terms alkyl, alkenyl, alkynyl, each of which being optionally substituted as set forth below. The term "heteroaliphatic" encompasses aliphatic groups in which at least one carbon atom in the carbon chain is substituted by a heteroatom such as O, S or N.

As used herein, an "alkyl" group refers to a saturated aliphatic hydrocarbon group containing from 1-20 (e.g., 2-18, 3-18, 1-8, 1-6, 1-4, or 1-3) carbon atoms. An alkyl group can be straight, branched, cyclic or any combination thereof. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents or can be multicyclic as set forth below. Unless specifically limited otherwise, the term "alkyl" as well as derivative terms such as "alkoxy" and "thioalkyl" as used herein, include within their scope, straight chain, branched chain and cyclic moieties.

As used herein, an "alkenyl" group refers to an aliphatic carbon group that contains from 2-20 (e.g., 2-18, 2-8, 2-6, or 2-4) carbon atoms and at least one double bond. Like an alkyl group, an alkenyl group can be straight, branched or cyclic or any combination thereof. Examples of an alkenyl group include, but are not limited to, allyl, isoprenyl, 2-butenyl, and 2-hexenyl. An alkenyl group can be optionally substituted with one or more substituents as set forth below.

As used herein, an "alkynyl" group refers to an aliphatic carbon group that contains from 2-20 (e.g., 2-8, 2-6, or 2-4) carbon atoms and has at least one triple bond. An alkynyl group can be straight, branched or cyclic or any combination thereof. Examples of an alkynyl group include, but are not limited to, propargyl and butynyl. An alkynyl group can be optionally substituted with one or more substituents as set forth below.

A "halogen" is an atom of the 17^{th} Group of the period table, which includes fluorine, chlorine, bromine and iodine.

As used herein, an "aryl" group (or *"aromatic"* group) used alone or as part of a larger moiety as in "aralkyl", "aralkoxy" or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyltetrahydrofluorenyl, or tetrahydroanthracenyl, anthracenyl) ring systems in which the mono cyclic ring system is aromatic or at least one of the rings in a bicyclic or tricyclic ring system is aromatic. The bicyclic and tricyclic groups include benzofused 2-3 membered carbocyclic rings. For example, a benzofused group includes phenyl fused with two or more C₄₋₈ carbocyclic moieties. An aryl is optionally substituted with one or more substituents as set forth below.

As used herein, an "aralkyl" or "arylalkyl" group refers to an alkyl group (e.g., a C₁₋₄ alkyl group) that is substituted with an aryl group. Both "alkyl" and "aryl" have been defined above. An example of an aralkyl group is benzyl. An aralkyl is optionally substituted with one or more substituents as set forth below.

As used herein, a "cycloalkyl" group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbomyl, cubyl, octahydroindenyl, decahydronaphthyl, bicyclo[3.2.1]octyl, bicyclo[2.2.2]octyl, bicyclo[3.3.1]-nonyl, bicyclo[3.3.2]decyl, bicyclo[2.2.2]octyl, adamantyl, azacycloalkyl, or ((amino carbonyl)cycloalkyl)cycloalkyl.

As used herein, the term "heteroaryl" group (or "heteroaromatic" group) refers to a monocyclic, bicyclic, or tricyclic ring system having 4 to 18 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. A heteroaryl group includes a benzofused ring system having 2 to 3 rings. For example, a benzofused group includes benzo fused with one or two 4 to 8 membered heterocycloaliphatic moieties (e.g. indolizyl, indolyl, isoindolyl, 3H-indolyl, indolinyl, benzo[b]furyl, benzo[b]thiophenyl, quinolinyl, or isoquinolinyl). Some examples ofheteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl, cinnolyl, phthalazyl, quinazolyl, quinoxalyl, isoquinolyl, 4H-quinolizyl, benzo-1,2,5-thiadiazolyl, or 1,8-naphthyridyl.

Without limitation, monocyclic heteroaryls include furyl, thiophenyl, 2H-pyrrolyl, pyrrolyl, oxazolyl, thazolyl, imidazolyl, pyrazolyl, isoxazolyl, isothiazolyl, 1,3,4-thiadiazolyl, 2H-pyranyl, 4H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo [b] furyl, benzo [b] thiophenyl, quinolinyl, isoquinolinyl, indolizyl, isoindolyl, indolyl, benzo [b] furyl, bexo[b] thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents as is set forth below.

A "heteroarylalkyl" group, as used herein, refers to an alkyl group (e.g., a C₁₋₄ alkyl group) that is substituted with a heteroaryl group. Both "alkyl" and "heteroaryl" have been defined above. A heteroarylalkyl is optionally substituted with one or more substituents as is set forth below.

As used herein, an "acyl" group refers to a formyl group or R-C(=O)- (such as alkyl-C(=O)-, also referred to as "alkylcarbonyl") where "alkyl" have been defined previously.

As used herein, the term "acyloxy" includes straight-chain acyloxy, branched-chain acyloxy, cycloacyloxy, cyclic acyloxy, heteroatom-unsubstituted acyloxy, heteroatom-substituted acyloxy, heteroatom-unsubstituted Cₙ-acyloxy, heteroatom-substituted Cₙ-acyloxy, alkylcarbonyloxy, arylcarbonyloxy, alkoxycarbonyloxy, aryloxycarbonyloxy, and carboxylate groups.

As used herein, an "alkoxy" group refers to an alkyl-O- group where "alkyl" has been defined previously.

As used herein, a "carboxy" group refers to -COOH, -COOR, -OC(=O)H, -OC(=O)R when used as a terminal group; or -OC(=O)- or -C(=O)O- when used as an internal group.

As used herein, "alkoxycarbonyl" means -COOR where R is alkyl as defined above, e.g. methoxycarbonyl, ethoxycarbonyl, and the like.

As used herein, a "sulfonyl" group refers to -S(=O)₂-R when used terminally and -S(=O)₂- when used internally.

The term "alkylthio" includes straight-chain alkylthio, branched-chain alkylthio, cycloalkylthio, cyclic alkylthio, heteroatom-unsubstituted alkylthio, heteroatom-substituted alkylthio, heteroatom-unsubstituted Cₙ-alkylthio, and heteroatom-substituted Cₙ-alkylthio. In certain embodiments, lower alkylthios are contemplated.

As used herein, the term "amine" or "amino" includes compounds where a nitrogen atom is covalently bonded to at least one carbon or heteroatom. The term "amine" or "amino" also includes -NH₂ and also includes substituted moieties. The term includes "alkyl amino" which comprises groups and compounds wherein the nitrogen is bound to at least one additional alkyl group. The term includes "dialkyl amino" groups wherein the nitrogen atom is bound to at least two additional independently selected alkyl groups. The term includes "arylamino" and "diarylamino" groups wherein the nitrogen is bound to at least one or two independently selected aryl groups, respectively.

The term "haloalkyl" refers to alkyl groups substituted with from one up to the maximum possible number of halogen atoms. The terms "haloalkoxy" and "halothioalkyl" refer to alkoxy and thioalkyl groups substituted with from one up to five halogen atoms.

The phrase "optionally substituted" is used interchangeably with the phrase "substituted or unsubstituted". As described herein, compounds of the invention can optionally be substituted with one or more substituents, such as are illustrated generally above, or as exemplified by particular classes, subclasses, and species of the invention. As described herein any of the above moieties or those introduced below can be optionally substituted with one or more substituents described herein. Each substituent of a specific group is further optionally substituted with one to three of halo, cyano, oxoalkoxy, hydroxy, amino, nitro, aryl, haloalkyl, and alkyl. For instance, an alkyl group can be substituted with alkylsulfanyl and the alkylsulfanyl can be optionally substituted with one to three of halo, cyano, oxoalkoxy, hydroxy, amino, nitro, aryl, haloalkyl, and alkyl.

In general, the term "substituted", whether preceded by the term "optionally" or not, refers to the replacement of hydrogen radicals in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

Modifications or derivatives of the compounds disclosed throughout this specification are contemplated as being useful with the methods and compositions of the invention. Derivatives may be prepared and the properties of such derivatives may be assayed for their desired properties by any method known to those of skill in the art. In certain aspects, "derivative" refers to a chemically modified compound that still retains the desired effects of the compound prior to the chemical modification.

### THE SENSITIZER COMPOUND

The composition according to the present invention comprises as component (i) at least one sensitizer compound that, upon exposure to electromagnetic radiation, changes from a ground state into an excited state, wherein the sensitizer compound is preferably selected from the group consisting of xanthones, thioxanthones, coumarines, anthracenes, acridines, imidazoles, triazines, amino benzoates and a mixture of at least two thereof and wherein thioxanthone and derivatives of thioxanthone are most preferred.

Preferably, the sensitizer compound (i) has a solubility in propylene glycol monomethyl ether acetate (PGMEA) of at least 0.05 wt.-%, preferably at least 0.5 wt.-%, more preferably at least 5 wt.-%, even more preferably at least 10 wt.-% and most preferably at least 50 wt.-%, the solubility being measured at 25°C.

According to a particularly preferred embodiment of the composition according to the present invention the sensitizer compound (i) has the general structure (II) 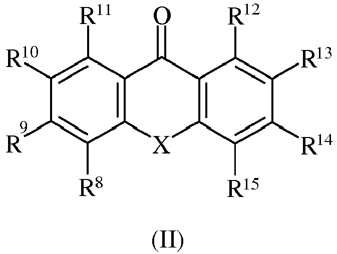 wherein R⁸, R⁹, R¹⁰, R¹¹, R¹², R¹³, R¹⁴ and R¹⁵ independently from each other represent a hydrogen atom, an organic residue, a hydroxy group, an amine group, a nitro group or a halogen, wherein two of these residues may form together an aliphatic or aromatic ring system, and X represents either O or S, preferably S.

Preferred substituents for R⁸, R⁹, R¹⁰, R¹¹, R¹², R¹³, R¹⁴ and R¹⁵ are selected from the group consisting of H, Cl, Br, I, -OH, -NO₂, -CN, an alkyl group such as -CH₃, -CH₂CH₃, -CH(CH₃)₂ or -C(CH₃)₃, an alkoxy group such as -O-CH₃, -O-CH₂CH₃ or -O-CH₂-CH₂-CH₃, an alkoxycarbonyl group such as -C(=O)-O-CH₃ and -C(=O)-O-CH₂CH₃, an acyl group such as -C(=O)CH₃, -C(=O)CH₂CH₃ or -C(=O)CH₂CH₂CH₃, an amine group such as -NH₂, -NHCH₃, -N(CH₃)₂, -N(CH₃)₂, -NH(CH₂CH₃), -N(CH₂CH₃)₂, -NH(CH₂CH₂OH) or -N(CH₂CH₂OH)₂, or a sulfonyl group such as -SO₂Ph and -O-CH₂-CH(OH)-CH₂-N⁺(CH₃)₃Cl⁻.

Particularly suitable compounds in this context are
- thioxanthone (the compound (i) of the general structure (II) in which R⁸, R⁹, R¹⁰, R¹¹, R¹², R¹³, R¹⁴ and R¹⁵ represent a hydrogen atom and X represents S);
- 2-isopropylthioxanthone (also called "2-ITX", i. e. the compound (i) of the general structure (II) in which R⁸, R⁹, R¹⁰, R¹¹, R¹², R¹⁴ and R¹⁵ represent a hydrogen atom, R¹³ represents -CH(CH₃)₂ and X represents S), 4-isopropylthioxanthone (also called "4-ITX", i. e. the compound (i) of the general structure (II) in which R⁸, R⁹, R¹⁰, R¹¹, R¹², R¹³ and R¹⁴ represent a hydrogen atom, R¹⁵ represents -CH(CH₃)₂ and X represents S) or an isomer mixture of 2-ITX and 4-ITX;
- 2,4-diethylthioxanthone (also called "2,4-DETX", the compound (i) of the general structure (II) in which R⁸, R⁹, R¹⁰, R¹¹, R¹² and R¹⁴ represent a hydrogen atom, R¹³ and R¹⁵ represent a -CH₂CH₃ group and X represents S);
- (2-hydroxy-3-(3,4-dimethyl-9-oxo-9H-thioxanthen-2-yloxy)-N,N,N-trimethyl-1-propanium chloride (also called "QTX", the compound (i) of the general structure (II) in which R⁸, R⁹, R¹⁰, R¹¹ and R¹² represent a hydrogen atom, R¹⁴ and R¹⁵ represent a -CH₃ group, R¹³ represents a -O-CH₂-CH(OH)-CH₂-N⁺(CH₃)₃Cl⁻ group and X represents S);
- 1-chloro-4-propoxythioxanthone (also called "CPTX", the compound (i) of the general structure (II) in which R⁸, R⁹, R¹⁰, R¹¹, R¹³ and R¹⁴ represent a hydrogen atom, R¹² represents a chlorine atom, R¹⁵ represent a -O-CH₂-CH₂-CH₃ group and X represents S);
- 2-chlorothioxanthone (also called "CTX", the compound (i) of the general structure (II) in which R⁸, R⁹, R¹⁰, R¹¹, R¹³, R¹⁴ and R¹⁵ represent a hydrogen atom, R¹² represents a chlorine atom and X represents S);
- thioxanthone-anthracene (also called TX-A, the compound (i) of the general structure (II) in which R⁸, R⁹, R¹⁰, R¹¹, R¹² and R¹⁵ represent a hydrogen atom, R¹³ and R¹⁴ are together part of a naphthalene group and X represents S) having the following structure:

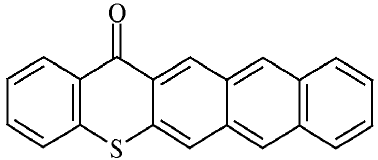

According to a further particularly preferred embodiment of the composition according to the present invention the sensitizer compound (i) has the general structure (III) 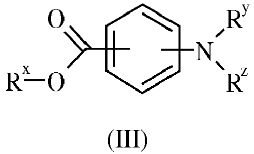 wherein R^{x} R^{y} and R^{z} independently from each other represent a hydrogen atom or an aliphatic or heteroaliphatic residue having 1 to 18 carbon atoms, wherein
- R^{y} and R^{z} independently from each other preferably represent an alkyl group, more preferably an alkyl group selected from the group consisting of such as -CH₃, -CH₂CH₃, -CH₂CH₂CH₃, -CH₂CH₂CH₂CH₃, -CH(CH₃)₂, -CH₂CH₂CH₂CH(CH₃)₂ (isoamyl), -CH₂-CH(CH₂CH₃)(CH₂CH₂CH₂CH₃) (2-ethylhexyl) and -C(CH₃)₃,
   and
- R^{x} preferably represents an alkyl group, more preferably an alkyl group selected from the group consisting of such as -CH₃, -CH₂CH₃, -CH₂CH₂CH₃, -CH₂CH₂CH₂CH₃, -CH(CH₃)₂, -CH₂CH₂CH₂CH(CH₃)₂ (isoamyl), -CH₂-CH(CH₂CH₃)(CH₂CH₂CH₂CH₃) (2-ethylhexyl) or an ether group, more preferably a group -CH₂CH₂-O-CH₂CH₂CH₂CH₃ (butoxyethyl).

Examples of suitable sensitizer compounds (i) having the general structure (III) are selected from the group consisting of methyl-4-dimethylaminobenzoate, ethyl-4-dimethylaminobenzoate, isoamyl-4-dimethylaminobenzoate, (n-butoxy)ethyl-4-dimethylaminobenzoate and 2-ethylhexyl 4-dimethylaminobenzoate.

### THE ACID GENERATING COMPOUND

The composition according to the present invention comprises as component (ii) at least one acid generating compound, the acid generating compound being an NIT derivative having the general structure (I). Preferably, residues R¹, R², R³, R⁴, R⁵ and R⁶ in general structure (I) are selected to ensure a sufficiently high solubility in organic solvents such as PGMEA. In this context it is particularly preferred that the solubility of the acid generating compound (ii) in PGMEA is at least 0.1 wt.-%, preferably at least 1.0 wt.-% and most preferably at least 10.0 wt.-%, the solubility being measured at 25°C.

### First particular embodiment of the acid generating compound (ii)

According to a first preferred embodiment of the acid generating compound (ii) this compound has the general structure (Ia) or (Ib) 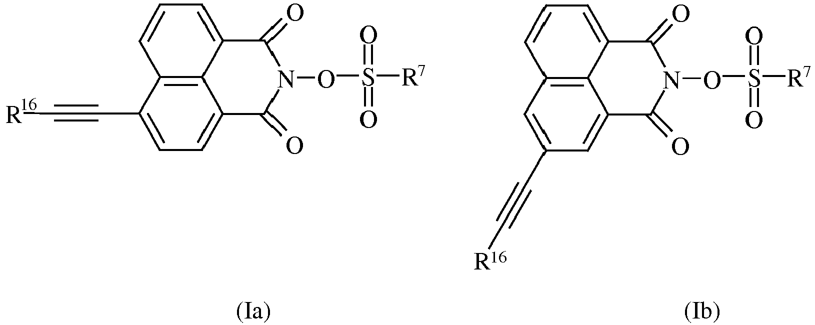 wherein
R¹⁶ is selected from the group consisting of
   - a hydrogen atom,
   - an aliphatic group having a carbon number of from 1 to 18 in which one or more hydrogen atoms may be substituted by a halogen atom,
      and
   - an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
R⁷ is selected from the group consisting of
   - -CH₃, -CH₂F, -CHF₂, or-CF₃,
   - an aliphatic group having a carbon number of from 2 to 18, which may be substituted by one or more halogen atom(s),
   - an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
   - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
      and
   - an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

According to a preferred variant of the first preferred embodiment of the acid generating compound (ii) having the general structure (Ia) or (Ib) R⁷ is -CF₃ and R¹⁶ is selected from the group consisting of
- a hydrogen atom,
- an aliphatic group having a carbon number of from 2 to 18 which comprises at least one moiety selected from the group consisting of -O-, -S-, -C(=O)-, -C(=O)-O-, -C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
- -CH₂CH(CH₃)₂, -CH₂CH=CHCH₃, or -CH₂CH₂CH=CH₂,
- a group represented by the Formula (A):

   -CH₂-R¹⁷ (A)

   wherein R¹⁷ is selected from the group consisiting of an aliphatic group having a carbon number of from 4 to 18,
- cyclopropenyl,
   and
- a group represented by the Formula (B): 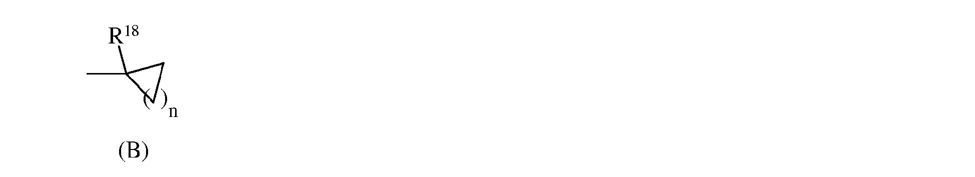 wherein R¹⁸ is selected from the group consisting of a hydrogen atom or an alkyl group having a carbon number of from 1 to 10; and n is equal to 1 to 5.

In some embodiments, R¹⁶ in Formulas (Ia) and (Ib) is an aliphatic group having a carbon number of from 1 to 18 in which one or more hydrogen atoms may be substituted by a halogen atom. Preferred examples include methyl, ethyl, propyl, isopropyl, butyl, isobutyl, sec-butyl, tert-butyl, n-pentyl, n-hexyl, 2-ethylhexyl, n-heptyl, n-octyl, n-nonyl, and n-decyl. In an embodiment, R¹⁶ in Formulas (Ia) and (Ib) is an aliphatic group having a carbon number of from 1 to 18 in which one or more hydrogen atoms may be substituted by a halogen atom and R⁷ is an aliphatic group having a carbon number of from 1 to 18, which may be substituted by one or more halogen atom(s). Preferably, R⁷ is an aliphatic group having a carbon number of from 1 to 6 and, more preferably, an aliphatic group having a carbon number of from 1 to 4, which is substituted by at least one fluorine atom. Examples of such acid generating compounds (ii) include those in Table 1:

**Table 1**

| | |
|---|---|
| 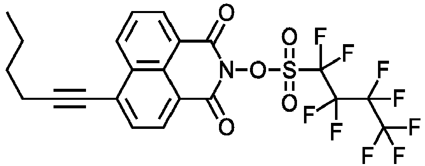 | T1 |
| 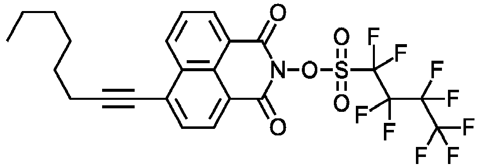 | T3 |
| 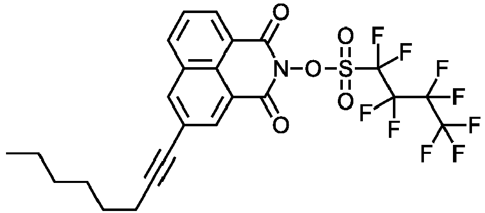 | T11 |

In other embodiments, R¹⁶ in Formulas (Ia) and (Ib) is an aliphatic group having a carbon number of from 2 to 18 which comprises at least one -C(=O)-O- moiety. In an embodiment, R¹⁶ in Formulas (Ia) and (Ib) is an aliphatic group having a carbon number of from 2 to 18 which comprises at least one -C(=O)-O- moiety and R⁷ is an aliphatic group having a carbon number of from 1 to 18, which may be substituted by one or more halogen atom(s). Preferably, R⁷ is an aliphatic group having a carbon number of from 1 to 6 and, more preferably, an aliphatic group having a carbon number of from 1 to 4, which is substituted by at least one fluorine atom. Examples of such acid generating compounds (ii) include those in Table 2:

**Table 2**

| | |
|---|---|
| 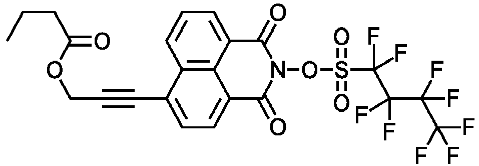 | T24 |
| 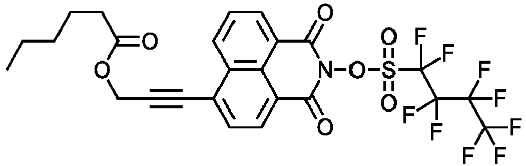 | T25 |

In yet other embodiments, R¹⁶ in Formulas (Ia) and (Ib) is an aliphatic group having a carbon number of from 1 to 18 in which one or more hydrogen atoms may be substituted by a halogen atom. In another embodiment, R¹⁶ in Formulas (Ia) and (Ib) is an aliphatic group having a carbon number of from 1 to 18 in which one or more hydrogen atoms may be substituted by a halogen atom and R⁷ is an aryl or heteroaryl group having a carbon number of from 4 to 18 in which one or more hydrogen atoms may be substituted by a halogen atom, an aliphatic group, or a haloalkyl group. Examples of such acid generating compounds (ii) include those in Table 3:

**Table 3**

| | |
|---|---|
| 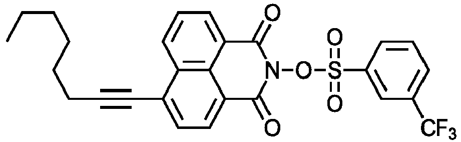 | T36 |
| 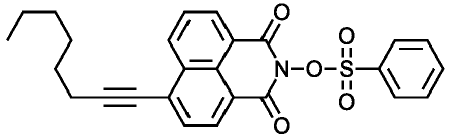 | T37 |

In the most preferred embodiments of Formulas (Ia) and (Ib) according to the invention, R⁷ is -CF₃. In such embodiments the above-recited proviso applies. Preferred embodiments include compounds where R¹⁶ is a group represented by the Formula (A):

-CH₂-R¹⁷ (A)

wherein R¹⁷ is selected from the group consisting of an aliphatic group having a carbon number of from 4 to 18. Examples include n-butyl, iso-butyl, sec-butyl, tert-butyl, n-pentyl, n-hexyl, 2-ethylhexyl, n-heptyl, n-octyl, n-nonyl, and n-decyl. Examples of such acid generating compounds (ii) include those in Table 4:

**Table 4**

| | |
|---|---|
| 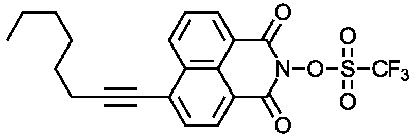 | T2 |
| 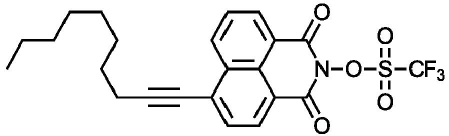 | T6 |
| 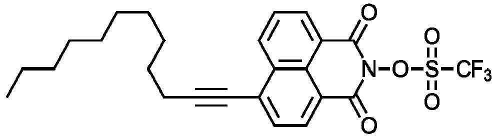 | T7 |
| 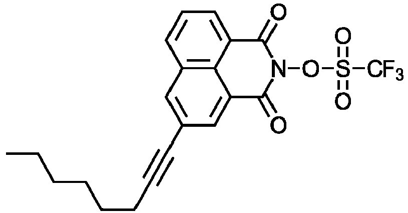 | T10 |
| 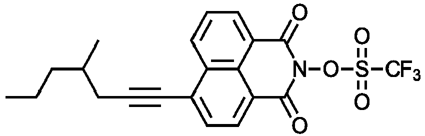 | T13 |

In other preferred embodiments where R⁷ is -CF₃ in Formulas (Ia) and (Ib), R¹⁶ is an aliphatic group having a carbon number of from 2 to 18 which comprises at least one -C(=O)-O- or -O-C(=O)- moiety, wherein the aliphatic group optionally comprises at least one halogen atom. Examples of such acid generating compounds (ii) include those in Table 5:

**Table 5**

| | |
|---|---|
| 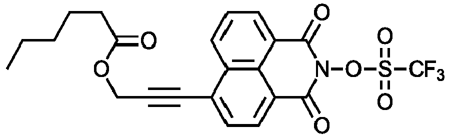 | T4 |
| 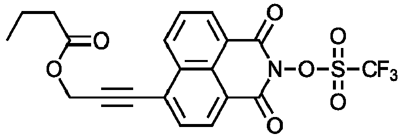 | T5 |
| 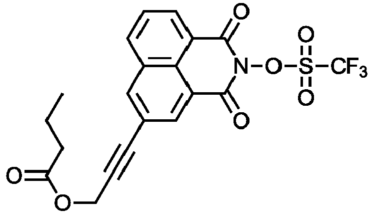 | T8 |
| 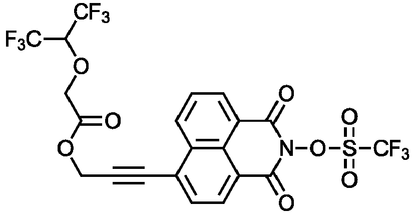 | T9 |
| 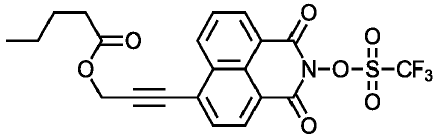 | T12 |
| 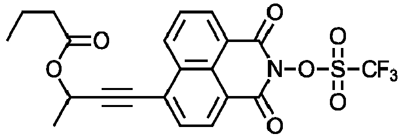 | T14 |
| 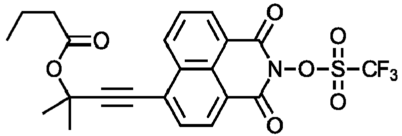 | T16 |
| 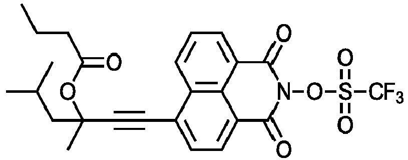 | T17 |
| 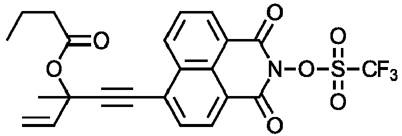 | T18 |
| 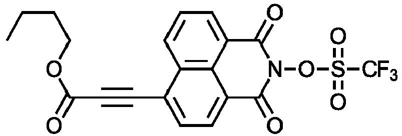 | T19 |
| 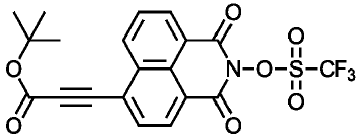 | T20 |
| 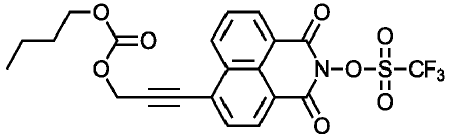 | T22 |
| 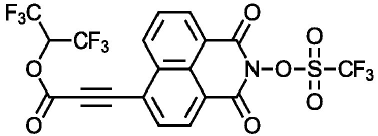 | T23 |
| 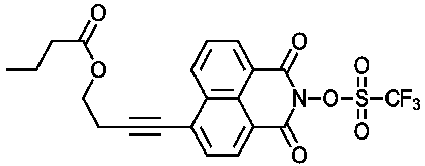 | T32 |
| 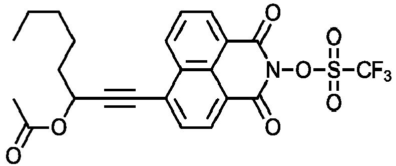 | T33 |
| 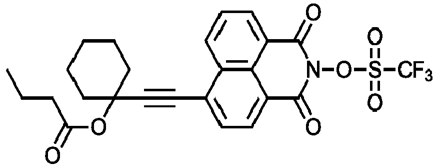 | T34 |
| 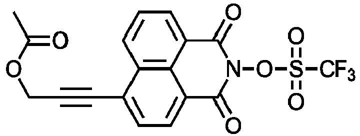 | T35 |

In yet other preferred embodiments where R⁷ is -CF₃ in Formulas (Ia) and (Ib), R¹⁶ is an aliphatic group having a carbon number of from 2 to 18 which comprises at least one -O-, -S-, or -C(=O)- moiety, wherein the aliphatic group optionally comprises at least one halogen atom. Examples of acid generating compounds (ii) include those in Table 6:

**Table 6**

| | |
|---|---|
| 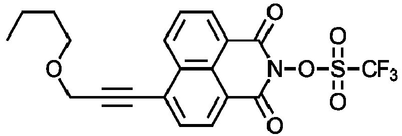 | T15 |
| 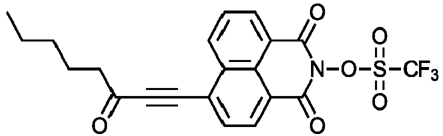 | T21 |
| 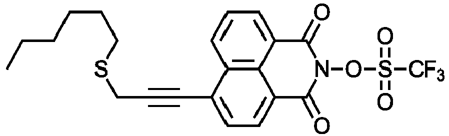 | T29 |

In yet other preferred embodiments where R is -CF₃ in Formulas (Ia) and (Ib), R¹⁶ is an aliphatic group having a carbon number of from 2 to 18 which comprises at least one -C(=O)-S-, -C(=O)-NH-, -O-C(=O)-NH-, -C(=O)-NR^{a}-, -O-C(=O)-NR^{a}-, or -C(=O)-NR^{a}R^{b} moiety, wherein R^{a} and R^{b} are as defined above, and wherein the aliphatic group optionally comprises at least one halogen atom. Examples of such acid generating compounds (ii) include those in Table 7:

**Table 7**

| | |
|---|---|
| 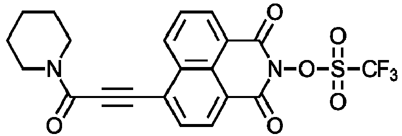 | T26 |
| 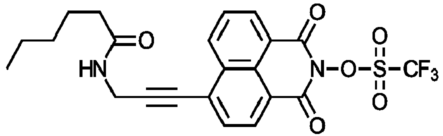 | T30 |
| 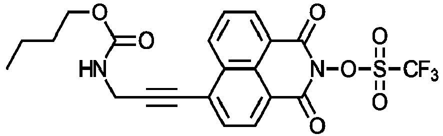 | T31 |
| 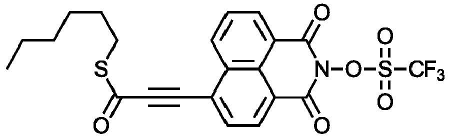 | T28 |

In another preferred embodiment where R⁷ is -CF₃ in Formulas (Ia) and (Ib), R¹⁶ is a group represented by the Formula (B): 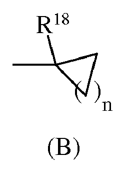 wherein R¹⁸ is selected from the group consisting of a hydrogen atom or an alkyl group having a carbon number of from 1 to 10; and n is equal to 1 to 5. An example of such an acid generating compound (ii) is compound T27

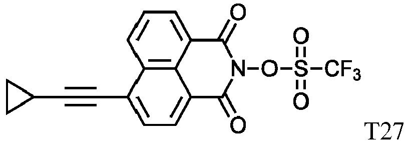

In other embodiments of the invention, both R⁷ and R¹⁶ in Formulas (Ia) and (Ib) are an aliphatic group having a carbon number of from 1 to 18. Examples of such acid generating compounds (ii) include compounds T38, T40 and T41:

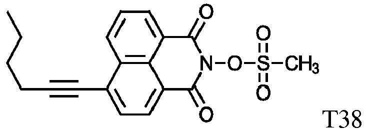

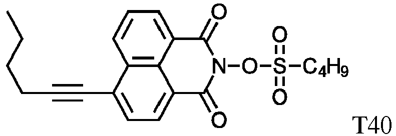

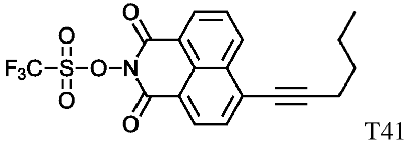

In other embodiments of the invention, R⁷ in Formulas (Ia) and (Ib) is an aliphatic group having a carbon number of from 2 to 18 which comprises at least one -C(=O)- moiety and R¹⁶ in Formulas (Ia) and (Ib) is an aliphatic group having a carbon number of from 1 to 18. Examples of such an acid generating compound (ii) include compound T39:

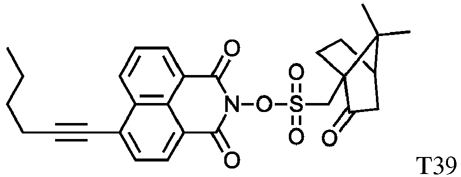

There is no particular limitation for the method for producing the acid generating compounds (ii) according to the above described first particular embodiment and any well-known approach can be used to make the compounds of Formulas (Ia) and (Ib). Two typical routes that we adopted to synthesize triflate compounds are illustrated in Scheme 1. Compounds with a triple-bond substituent at 3 position can be similarly synthesized by starting from 3-bromo anhydride. The starting anhydrides (4-bromo-1,8-naphthalic anhydride and 3-bromo-1,8-naphthalic anhydride) are commercially available.

As shown in Scheme 1, Sonagashira coupling between 4-bromo-1,8-naphthalic anhydride and a terminal alkyne affords naphthalic anhydrides with a triple-bond substituent. These anhydrides are converted to the final N-hydroxynaphthalimide sulfonate derivatives via two different approaches. The first approach involves an one-pot reaction using 2.2 equivalents of triflic anhydride. The second approach allows for the isolation of N-hydroxyl imide intermediates and only 1.2 equivalent of triflic anhydride is required.

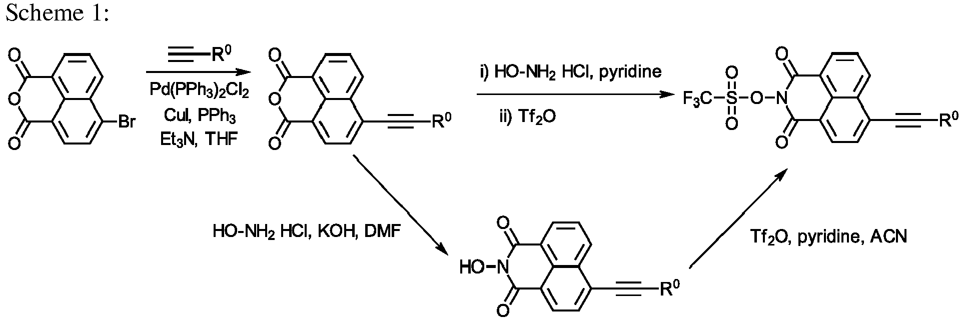

### Second particular embodiment of the acid generating compound (ii)

According to a second preferred embodiment of the acid generating compound (ii) this compound has the general structure (Ic) or (Id) 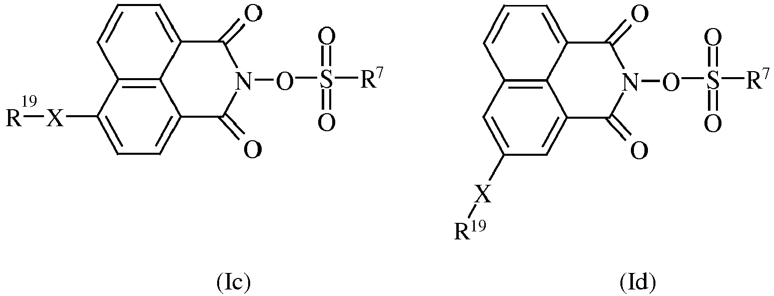 wherein
X is O or S,
R¹⁹ is selected from the group consisting of
   - an aliphatic group having a carbon number of from 1 to 18, preferably 1 to 3, in which one or more hydrogen atoms may be substituted by a halogen atom,
   - an aliphatic group having a carbon number of from 2 to 18 which comprises at least one moiety selected from the group consisting of -S-, -C(=O)-S-, -O-S(=O)₂-, -O-C(=O)-O-, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
   - a group represented by the Formula (C):

      -R²⁰-Ar (C)

      wherein
      - R²⁰ is a single bond or an aliphatic group having a carbon number of from 1 to 20, which may comprise at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
         and
      - Ar is an aryl or heteroaryl group in which one or more hydrogen atoms may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
   - a group represented by the Formula (D):

      -R²¹-R²²-O-C(=O)-R²³-R²⁴ (D)

      wherein
      - R²¹ and R²² are each independently a aliphatic group having a carbon number of from 1 to 5,
      - R²³ is an aliphatic group having a carbon number of from 1 to 10;
         and
      - R²⁴ is an aliphatic group having a carbon number of from 1 to 18, which comprises at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, and wherein the at least one moiety, if more than one, may be separated by an aliphatic group,
      and
   - a group represented by the Formula (E):

      -R²⁵-C(=O)-O-R²⁶ (E)

      wherein
      - R²⁵ is an aliphatic group having a carbon number of from 2 to 18, which may comprise at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
         and
      - R²⁶ is an aliphatic group having a carbon number of from 1 to 18 which comprises at least one moiety selected from the group consisting of -O-, -S-, -C(=O)-O-,-C(=O)-S-, -O-S(=O)₂-, -O-C(=O)-O-, -C(=O)-NH-, -O-C(=O)-NH-, -C(=O)-NR^{a}-, -O-C(=O)-NR^{a}- and -C(=O)-NR^{a}R^{b}, wherein R^{a} and R^{b} are as defined above, and wherein the at least one moiety, if more than one, may be separated by a aliphatic group,
R⁷ is selected from the group consisting of
   - an aliphatic group having a carbon number of from 1 to 18, which may be substituted by one or more halogen atom(s),
   - an aliphatic group having a carbon number of from 3 to 18 which comprises at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}- and -C(=O)-NR^{a}R^{b}, wherein R^{a} and R^{b} are as defined above, wherein the aliphatic group optionally comprises at least one halogen atom,
   - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group
      and
   - an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

In some preferred embodiments, R¹⁹ in formulas (Ic) and (Id) is an aliphatic group having from 1 to 18, more preferably 1 to 3 carbon atoms. Preferred examples include methyl, propyl, isopropyl, allyl, propargyl, cyclopropyl, propenyl, propynyl, and ethynyl. In other preferred embodiments, R¹⁹ in formulas (Ic) and (Id) is an aliphatic group having from 1 to 18, more preferably 1 to 3 carbon atoms and R⁷ in formulas (Ic) and (Id) is an aliphatic group having from a carbon number of from 1 to 18, and preferably from 1 to 6, which may be substituted by one or more halogen atom(s). Examples of such acid generating compounds (ii) include those in Table 8:

**Table 8**

| | |
|---|---|
| 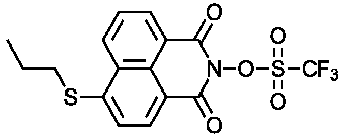 | S-1 |
| 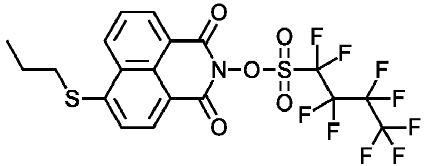 | S-2 |
| 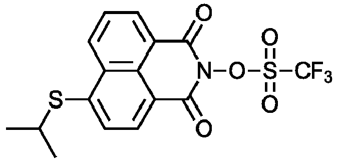 | S-3 |
| 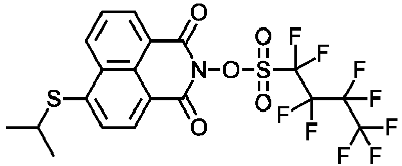 | S-4 |
| 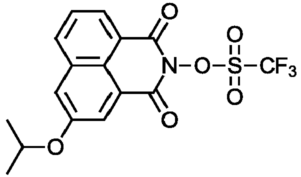 | O-29 |
| 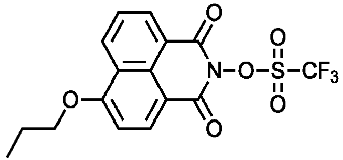 | O-30 |
| 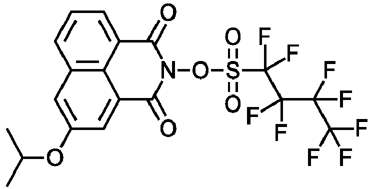 | O-45 |
| 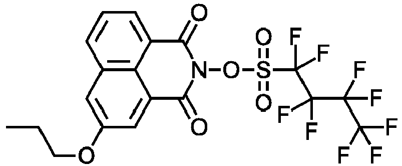 | O-46 |
| 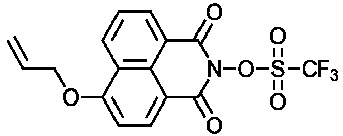 | O-47 |
| 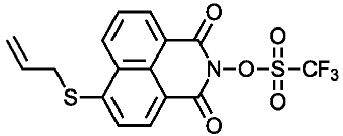 | O-48 |
| 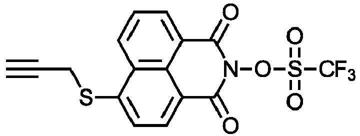 | O-49 |
| 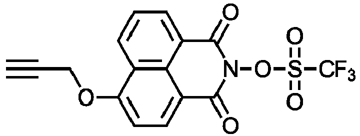 | O-50 |
| 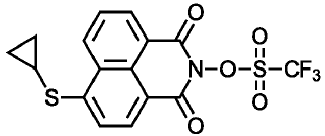 | O-51 |
| 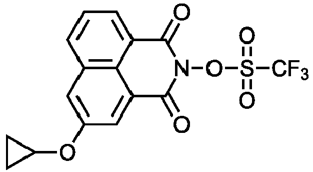 | O-52 |

In other preferred embodiments, R¹⁹ in formulas (Ic) and (Id) is an aliphatic group having a carbon number of from 2 to 18 which comprises at least one moiety selected from the group consisting of -S-, -C(=O)-S-, -O-S(=O)₂-, -O-C(=O)-O-, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom; and R⁷ in formulas (Ic) and (Id) is an aliphatic group having from a carbon number of from 1 to 18, and preferably from 1 to 6, which may be substituted by one or more halogen atom(s). Examples of such acid generating compounds (ii) include those in Table 9:

**Table 9**

| | |
|---|---|
| 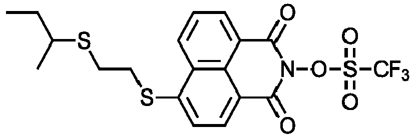 | S-15 |
| 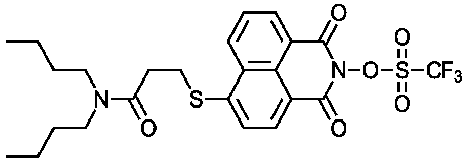 | S-17 |
| 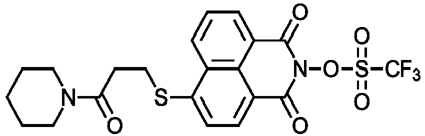 | S-18 |
| 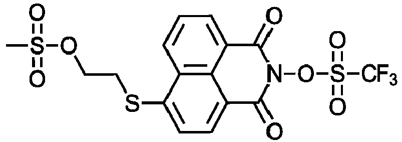 | S-22 |
| 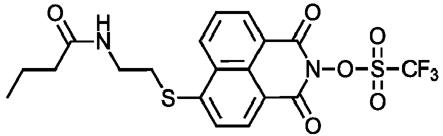 | S-23 |
| 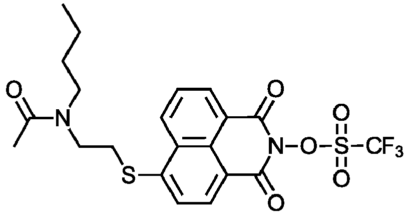 | S-24 |

In other preferred embodiments, R¹⁹ in formulas (Ic) and (Id) is a group represented by the formula (E):

-R²⁵-C(=O)-O-R²⁶ (E)

wherein R²⁵ is an aliphatic group having a carbon number of from 2 to 18, which may comprise at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group, and R²⁶ is an aliphatic group having a carbon number of from 1 to 18 which comprises at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, and wherein the at least one moiety, if more than one, may be separated by a aliphatic group, and R⁷ in formulas (Ic) and (Id) is an aliphatic group having from a carbon number of from 1 to 18, and preferably from 1 to 6, which may be substituted by one or more halogen atom(s). Examples of such acid generating compounds (ii) include those in in Table 10:

**Table 10**

| | |
|---|---|
| 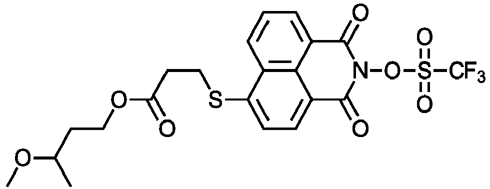 | S-11 |
| 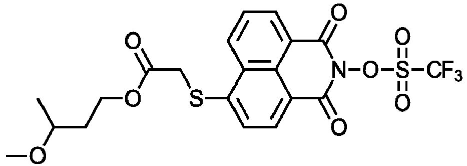 | S-16 |
| 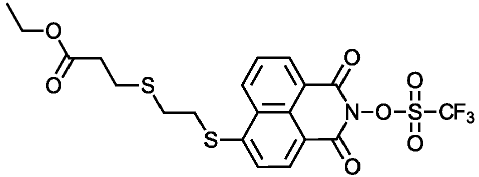 | S-34 |
| 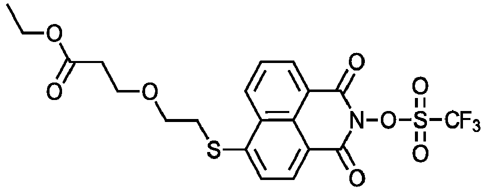 | S-35 |
| 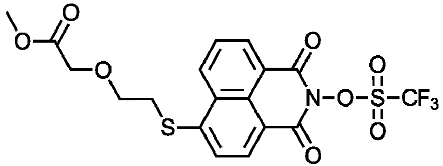 | S-36 |
| 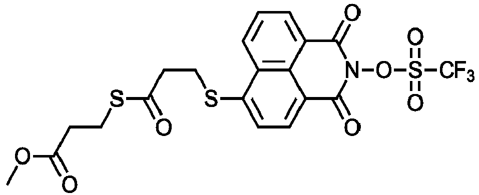 | S-37 |

In yet other preferred embodiments, R¹⁹ in formulas (Ic) and (Id) is a group represented by the formula (A):

-CH₂-R²⁰ (A)

wherein R²⁰ is a single bond or an aliphatic group having a carbon number of from 1 to 20, which may comprise at least one moiety selected from the group consisting of -O-, -S-, -C(=O)-O-, -C(=O)-S-, -O-S(=O)₂- -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group, and Ar is an aryl or heteroaryl group in which one or more hydrogen atoms may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group, and R⁷ in formulas (Ic) and (Id) is an aliphatic group having from a carbon number of from 1 to 18, and preferably from 1 to 6, which may be substituted by one or more halogen atom(s). Examples of such acid generating compounds (ii) include those in in Table 11:

**Table 11**

| | |
|---|---|
| 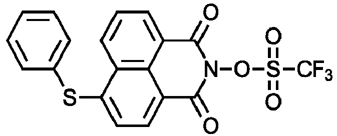 | S-5 |
| 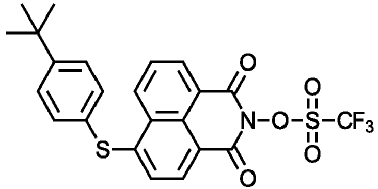 | S-6 |
| 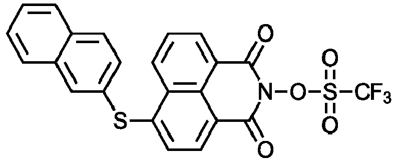 | S-7 |
| 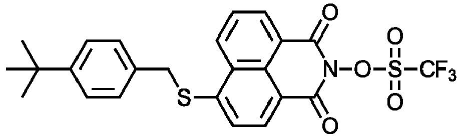 | S-8 |
| 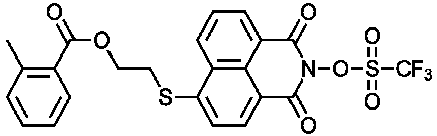 | S-9 |
| 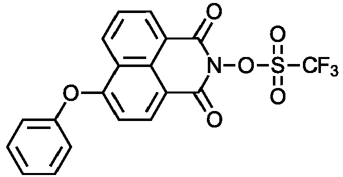 | O-12 |
| 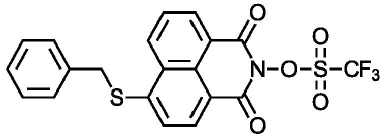 | S-13 |
| 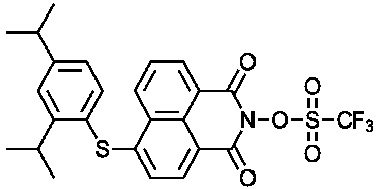 | S-14 |
| 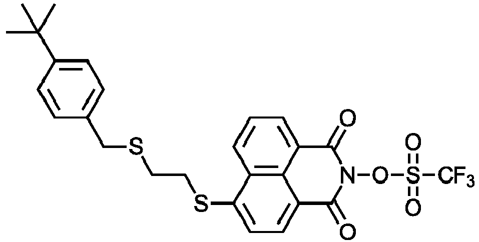 | S-19 |
| 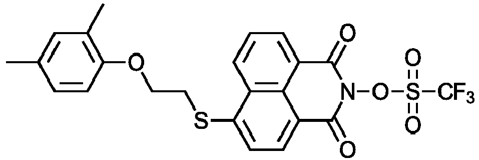 | S-20 |
| 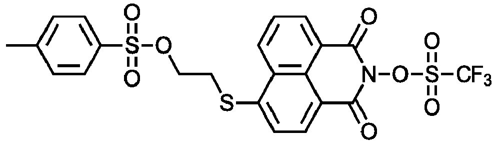 | S-21 |
| 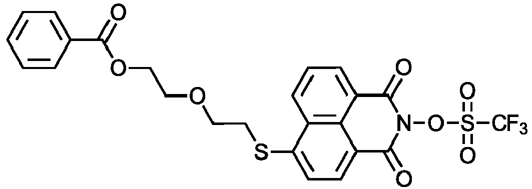 | S-39 |
| 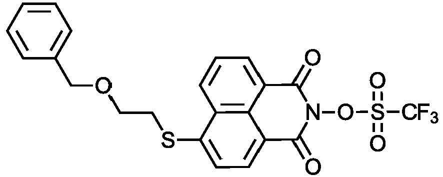 | S-40 |
| 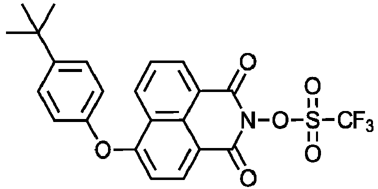 | O-41 |
| 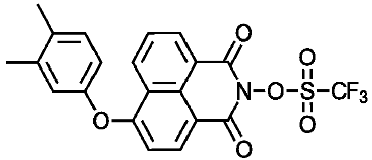 | O-42 |
| 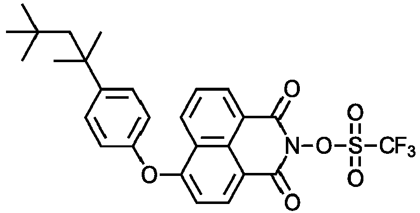 | O-44 |

In preferred embodiments, R²⁰ is an aliphatic group having a carbon number of from 1 to 20, which may comprise at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group.

In still yet other preferred embodiments, R⁷ in formulas (Ic) and (Id) is an aliphatic group having a carbon number of from 3 to 18 which comprises least one moiety selected from the group consisting of -O-, -S-, -C(=O)-O-, -C(=O)-S-, -O-S(=O)₂-, -O-C(=O)-O-, -C(=O)NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group comprises a bicyclic moiety or R⁷ in formulas (Ic) and (Id) is an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group; and R¹⁹ can be any group as defined above. Examples of such acid generating compounds (ii) include those in in Table 12:

**Table 12**

| | |
|---|---|
| 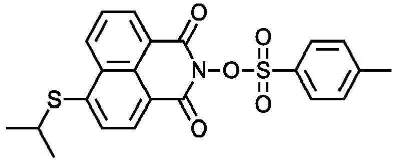 | S-10 |
| 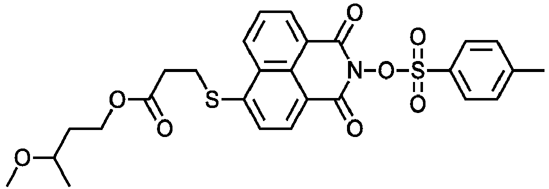 | S-43 |
| 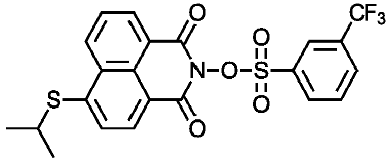 | S-25 |
| 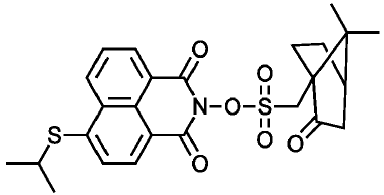 | S-33 |

In certain preferred embodiments of the invention in the acid generating compound (ii) represented by either formula (Ic) or formula (Id) X is an oxygen (0) or a sulfur (S) atom, R¹⁹ is a aliphatic group having a carbon number of from 1 to 3 in which one or more hydrogen atoms may be substituted by a halogen atom; and R⁷ is a aliphatic group having a carbon number of from 1 to 18, which may be substituted by one or more halogen atom(s).

In such embodiments, for example, X is S, R¹⁹ is propyl or isopropyl, and R⁷ is -CF₃ or -C₄F₉. In other embodiments, for example, X is O, R¹⁹ is propyl or isopropyl, and R⁷ is -CF₃ or -C₄F₉. In more preferred embodiments, R¹⁹ is isopropyl. Examples of such acid generating compounds (ii) include those in in Table 13:

**Table 13**

| | |
|---|---|
| 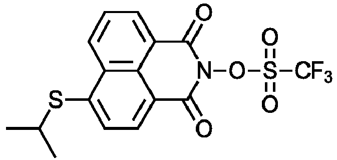 | S-3 |
| 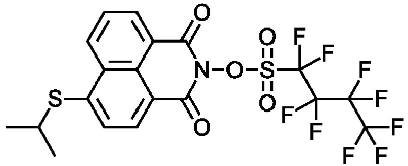 | S-4 |
| 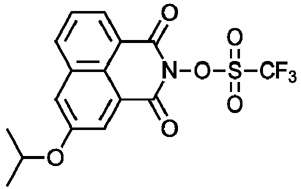 | O-29 |
| 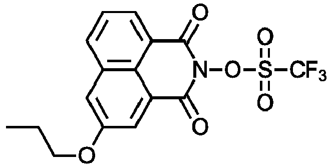 | O-30 |
| 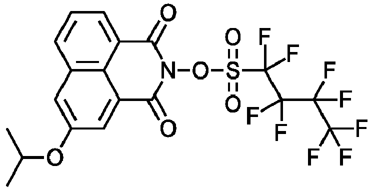 | O-45 |
| 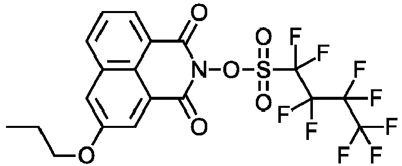 | O-46 |

There is also no particular limitation for the method for producing the acid generating compounds (ii) according to the above described second particular embodiment and any well-known approach can be used to make the compounds of Formulas (Ic) and (Id). Some examples of typical routes for synthesizing 4-substituted compounds are illustrated in Schemes 2 and 3, respectively. Compounds with substituents at 3 position can be similarly synthesized by starting from 3-substituted anhydrides. The starting anhydrides (4-bromo-1,8-naphthalic anhydride, 3-bromo-1,8-naphthalic anhydride, 4-hydroxy-1,8-naphthalic anhydride, and 3-hydroxy-1,8-naphthalic anhydride) are commercially available.

As shown in Scheme 2, compounds with alkylthio substituents can be readily synthesized from 4-bromo-1,8-naphthalic anhydride. Compounds with arylthio or aryloxy substituents can be similarly synthesized. Other organic bases (e.g., Et₃N and DABCO) besides DBU also work well for condition (a). NaOH can be used instead of KOH in condition (c). Compounds with alkyloxy substituents can be synthesized according to Scheme 3. 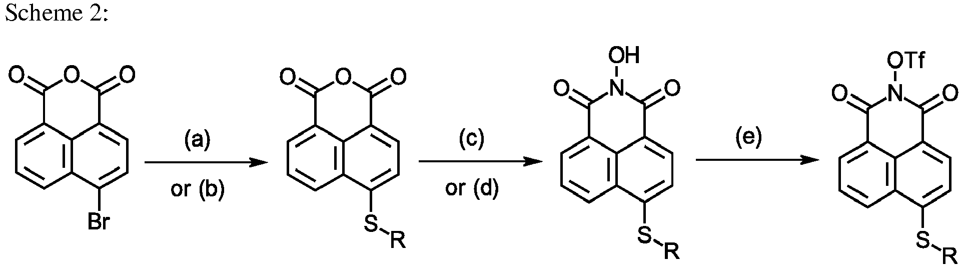 Conditions: (a) RSH, DBU, DMF; (b) RSH, K₂CO₃, DMF; (c) HO-NH_{2·}HCl, KOH, DMF; (d) HO-NH₂·HCl, pyridine; (e) Tf₂O, pyridine, ACN. 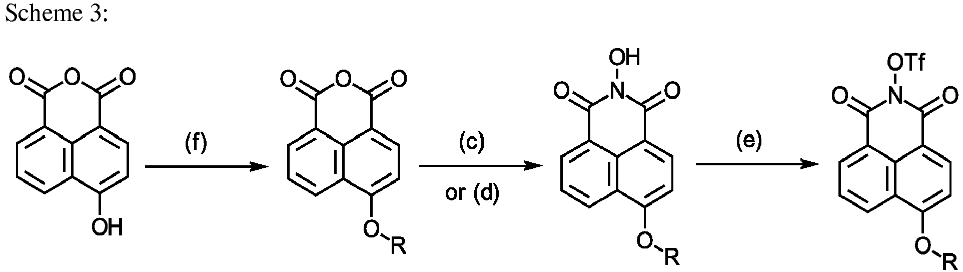 Conditions: (f) RBr, Cs₂CO₃, DMF.

### Third particular embodiment of the acid generating compound (ii)

According to a third preferred embodiment of the acid generating compound (ii) this compound has the general structure (I) in which at least one of R¹, R², R³, R⁴, R⁵ and R⁶ represents a group of the Formula (Fa) or (Fb)

-NR²⁷R²⁸ (Fa)

-A-NR²⁷R²⁸ (Fb)

wherein
R²⁷ and R²⁸ independently from each other represent
   - a hydrogen atom,
   - a linear or branched, optionally substituted aliphatic or heteroaliphatic group having 1-20 carbon atoms, in particular an alkyl group having 1-20 carbon atoms, preferably 1-10 carbon atoms and most preferably 1-5 carbon atoms, an optionally substituted cycloalkyl group having 5-20 carbon atoms, preferably 5-15 carbon atoms and most preferably 5-10 carbon atoms, or a linear or branched, optionally substituted alkyl carbonyl group having 2-20 carbon atoms, preferably 2-15 carbon atoms and most preferably 2-10 carbon atoms,
   or
R²⁷ and R²⁸ together form a heterocyclic group that, in addition to the nitrogen atom shown in Formula (Fa) or (Fb), optionally comprises a further heteroatom selected from the group consisiting of N, O and S,
A represents
   - an optionally substituted divalent aliphatic or heteroaliphatic group having 1-20 carbon atoms, in particular a C₁-C₂₀ alkylene group, preferably an optionally substituted C₁-C₁₀ alkylene group and most preferably an optionally substituted C₁-C₅ alkylene group, an optionally substituted C₁-C₂₀ oxyalkylene group, preferably an optionally substituted C₁-C₁₀ oxyalkylene group and most preferably an optionally substituted C₁-C₅ oxyalkylene group, a carbonyl group, a C₂-C₂₀ alkylene carbonyl group, preferably C₂-C₁₀ alkylene carbonyl an most preferably a C₂-C₅ alkylene carbonyl, or a group selected from the group consisting of *-S-(CH₂)ₙ- and *-S-(CH₂)ₙ-CO-, in which * indicates the bond to the aromatic ring in general structure (I) and n is an integer from 1 to 20, preferably a *-S-(CH₂)₂- group or a *-S-(CH₂)₂CO- group,
      or
   - a divalent aromatic or heteroaromatic group having 6-20 carbon atoms.

In photoresist compositions described in the prior art it is usually necessary to prevent acid generated in the exposed area during the course of a photolithography process (i. e. between the exposure with irradiation and the above mentioned *"post exposure bake*") from diffusing or migrating to the unexposed area so as to improve the resolution of the resulting pattern and to obtain a superior profile. In order to prevent the acid diffusion, an alkaline compound has been added to a chemical amplification type photoresist composition as a quencher for the acid in an amount ranging from 5 to 30 % per mole of the photoacid generator. A variety of alkaline compounds have been reported for use in photoresist compositions (see, for example, US-A-2011/0223535, US 7,479,361, US-A-2012/0077120, US-A-2003/0082481, 7,534,554 or US 7,592,126).

However, in the acid generating compound (ii) having the general structure (I) in which at least one of R¹, R², R³, R⁴, R⁵ and R⁶ represents a basic functional group of the Formula (Fa) or (Fb) a structural element that is capable of releasing an acid upon exposure to electromagnetic radiation (which in the photoresist compositions of the prior art was part of the photoacid generator compound) and a structural element that comprises at least one basic functional group (which in the photoresist compositions of the prior art was part of the separately added alkaline compound serving as a quencher) are combined within one compound. Such an acid generating compound (ii), when used in a photoacid composition, allows the production of a pattern with a higher resolution compared to a photoresist composition in which the photoacid generating compound and the alkaline compound are added as separate components and - at the same time - is characterized by a sensitivity towards electromagnetic radiation, in particular towards electromagnetic radiation with a wavelength of 200 to 500 nm, more particularly in the range from 300 to 450 nm, more particularly in the range from 350 to 440 nm and even more particularly towards electromagnetic radiation with a wavelength of 365 nm (i-line), that is comparable with the sensitivity of a photoresist composition that is free of any quencher compound.

Particularly preferred basic functional groups having the general formula (Fa) and (Fb) are those functional groups having a general formula selected from the group consisting of formulae (IVa) to (IVp) as shown in table 14 (the hydrogen atoms in the rings of formula (IVe) to (IVp) are not shown for clarity):

**Table 14**

| | |
|---|---|
| 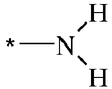 | IVa |
| 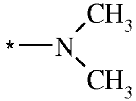 | IVb |
| 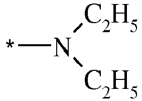 | IVc |
| 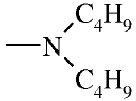 | IVd |
| 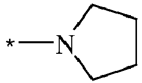 | IVe |
| 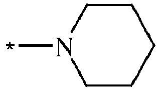 | IVf |
| 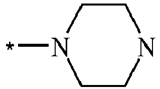 | IVg |
| 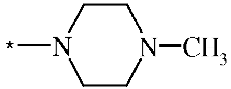 | IVh |
| 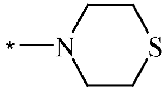 | IVi |
| 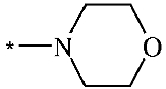 | IVj |
| 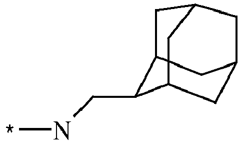 | IVk |
| 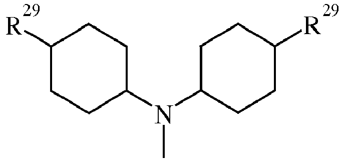 | IVl |
| 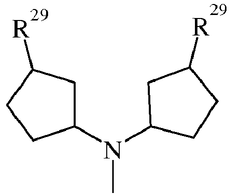 | IVm |
| 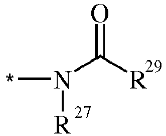 | IVn |
| 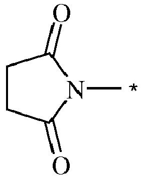 | IVo |
| 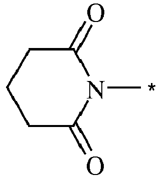 | IVp |

wherein R²⁷ is as defined in connection with the general formula (Fa) and (Fb) and R²⁹ represents a hydrogen or an aliphatic group having 1-20 carbon atoms.

In the third particular embodiment of the acid generating compound (ii) R⁷ is preferably selected from the group consisting of an optionally substituted, linear or branched alkyl group having 1-18 carbon atoms, an optionally substituted cycloalkyl group having 5-18 carbon atoms, a linear or branched fluorinated of perfluorinated alkyl group having 1-18 carbon atoms, a monovalent hydrocarbon group possessing a bicyclo ring having 7-15 carbon atoms, or an aryl group having 6-12 carbon atoms.

Suitable examples of residues R⁷ are all the groups that are bond to the nitrogen atom of the imide group in compounds 1 to 78 on pages 7 to 14 in US 2012/0289697 A1. The disclosure of US 2012/0289697 A1 with respect to the organic residues "R⁰⁷" that can be bond to the sulfur atom in the functional group -O-S(=O)₂-R in the structural formula (I) of US 2012/0289697 A1 is incorporated herein by reference.

It is particularly preferred that in the third particular embodiment of the acid generating compound (ii) that R⁷ represents a linear, branched or cyclic perfluoroalkyl group having the general formula -CₓF_{y}, in which x is an integer in the range from 1-8 and y is an integer in the range from 3-17. Examples of such residues R¹ are -C₆F₅ (pentafluorophenyl), -C₇F₇, -CF₃,-C₂F₅, -C₃F₇ and -C₄F₉.

In connection with the third particular embodiment of the acid generating compound (ii) it is furthermore preferred that the acid generating compound (ii) has the general structure (Ie), (If), (Ig) or (Ih) 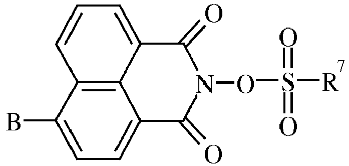 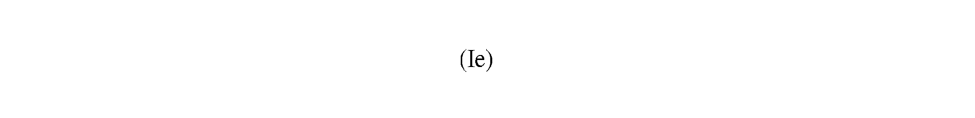 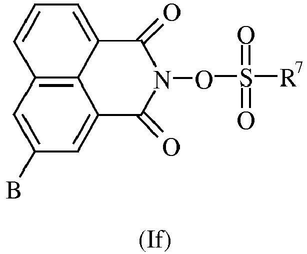 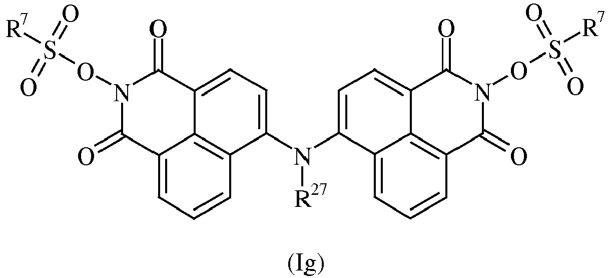 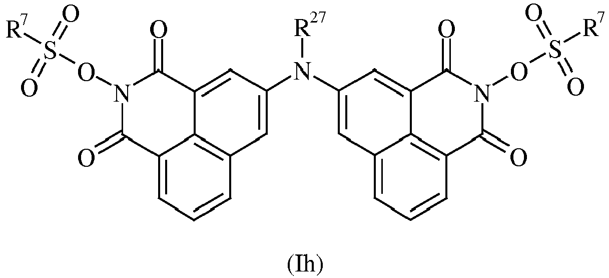 wherein B is a group of the Formula (Fa) or (Fb) and R⁷ and R²⁷ are as defined above.

Examples of suitable acid generating compound (ii) having the general structure (I) in which at least one of R¹, R², R³, R⁴, R⁵ and R⁶ represents a group of the Formula (Fa) or (Fb) can be selected from the group consisting of formulae (Va) to (Vr) as shown in table 15 (hydrogen atoms are not shown for clarity):

**Table 15**

| | |
|---|---|
| 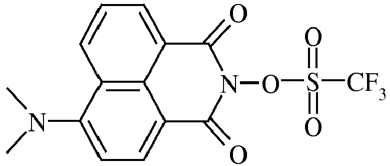 | Va |
| 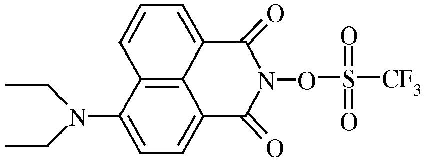 | Vb |
| 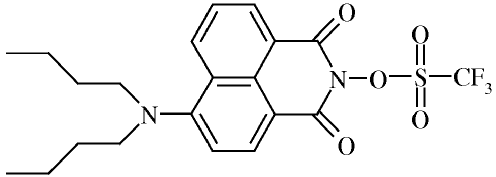 | Vc |
| 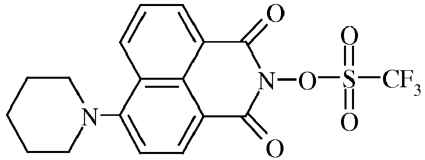 | Vd |
| 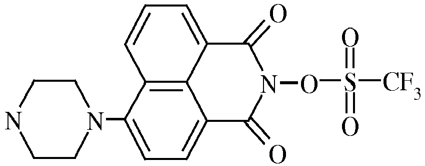 | Ve |
| 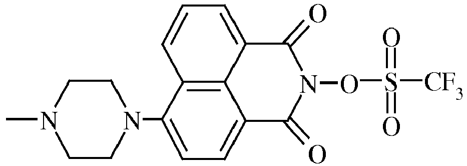 | Vf |
| 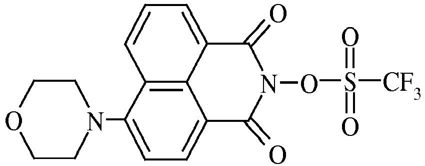 | Vg |
| 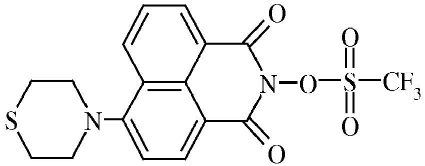 | Vh |
| 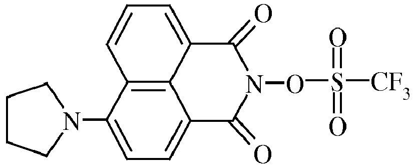 | Vi |
| 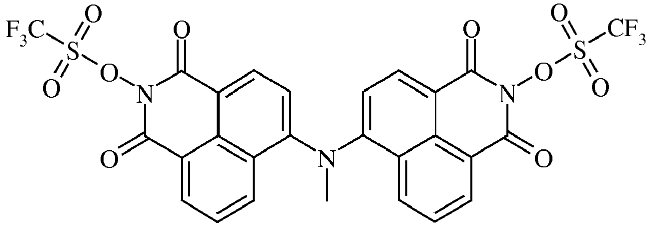 | Vj |
| 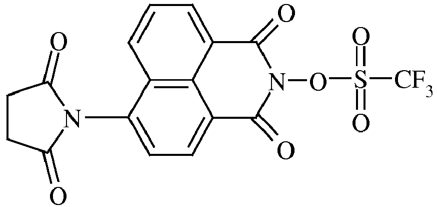 | Vk |
| 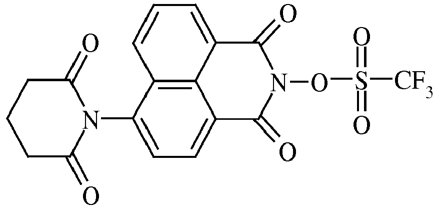 | Vl |
| 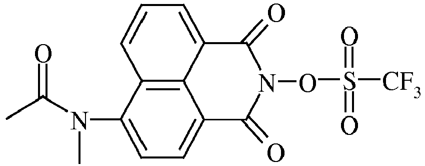 | Vm |
| 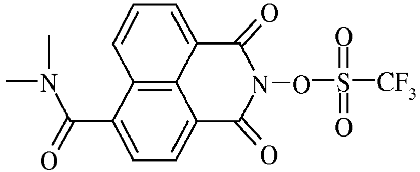 | Vn |
| 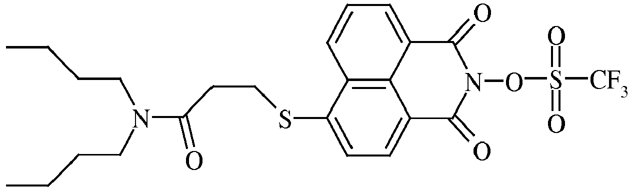 | Vo |
| 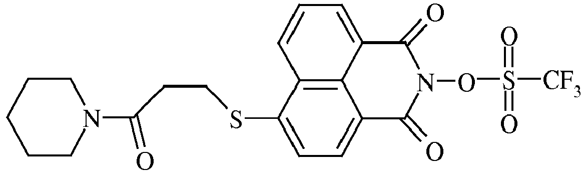 | Vp |
| 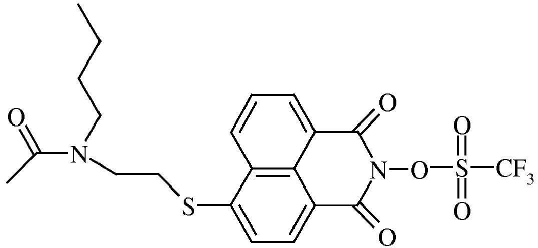 | Vq |
| 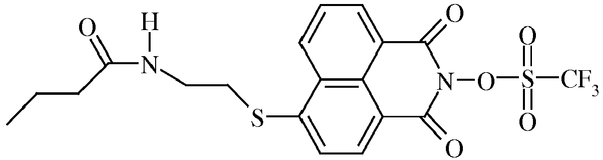 | Vr |

Also suitable are the corresponding compounds of the general formula (If) and (Ih) in which the basic functional group -NR²⁷R²⁸ (or -ANR²⁷R²⁸, respectively) is bond to the aromatic ring system at position 3 (instead of position 4 as shown in formulae (Va) to (Vk)).

Compounds of the general formula (Ie) and (If) can be prepared by reacting 3-substituted anhydrides such as 4-bromo-1,8-napthalic anhydride, 3-bromo-1,8-naphatlic anhydride, 4-hydroxy-1,8-napthalic anhydride or 3-hydroxy-1,8-naphatlic anhydride with the corresponding amine HNR²⁷R²⁸), followed by a reaction with hydroxylamine to form the N-hydroxyimide. In a final process step the thus obtained N-hydroxyimide is reacted with sulfonyl halogenates such as sulfonyl chlorides (R⁷-S(=O)₂-Cl) as shown in the following reaction scheme 4:

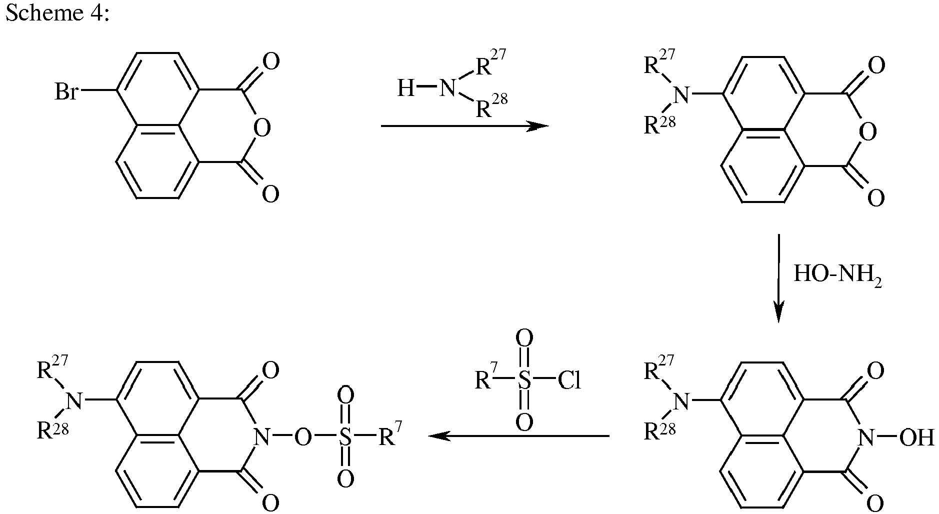

Further examples of suitable acid generating compounds (ii) having the general structure (I) in which at least one of R¹, R², R³, R⁴, R⁵ and R⁶ represents a group of the Formula (Fa) or (Fb) are compounds selected from the group consisting of having the general formula (Vs) to (Vv) as shown in table 16, in which R³⁰ and R³¹ are as defined for R²⁷ and R²⁸, X and Y are as defined for A and n is an integer in the range from 1 to 20:

**Table 16**

| | |
|---|---|
| 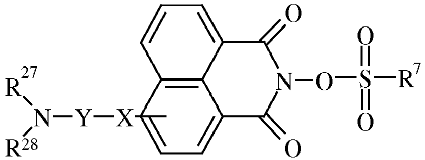 | Vs |
| 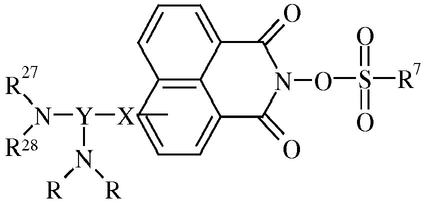 | Vt |
| 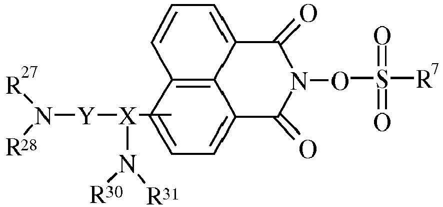 | Vu |
| 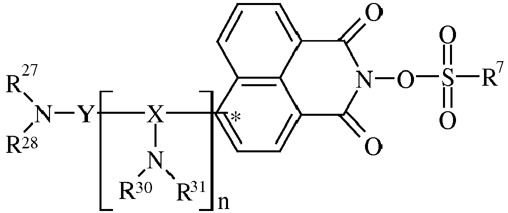 | Vv |

### Fourth particular embodiment of the acid generating compound (ii)

According to a fourth preferred embodiment of the acid generating compound (ii) this compound has the general structure (Ii) or (Ij) 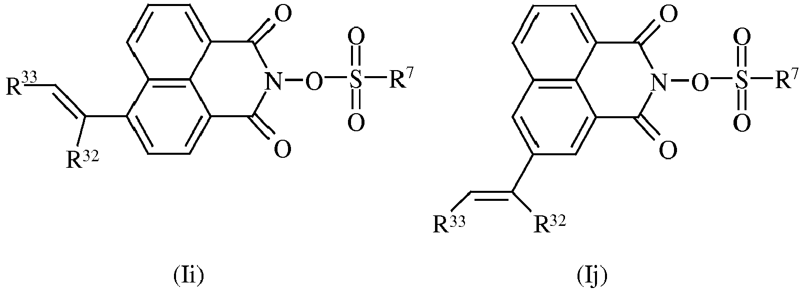 wherein
R³² and R³³ may be the same or different, may be connected to from an alicylic or heterocyclic group and are independently selected from the group consisting of
   - a hydrogen atom,
   - a cyano group,
   - an aliphatic, alicyclic or heterocyclic group having a carbon number of from 1 to 18 in which one or more hydrogen atoms may be substituted by a halogen atom,
   - an aliphatic group having a carbon number of from 1 to 18 which comprises at least one moiety selected from the group consisting of -O-, -S-, -C(=O) -, -C(=O)-O-, -C(=O)-S-, -O-C(=O)-O-, -CN, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic or heterocyclic group, and wherein the aliphatic group optionally comprises at least one halogen atom,
      and
   - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
R⁷ is selected from the group consisting of
   - an aliphatic, alicyclic or heterocyclic group having a carbon number of from 1 to 18, which may be substituted by one or more halogen atom(s),
   - an aliphatic or alicyclic group having a carbon number of from 1 to 18 which comprises at least one moiety selected from the group consisting of -O-, -S-, -C(=O), -C(=O)-O-, -C(=O)-S-, -O-C(=O)-O-, -CN,-C(=O)-NH-, -O-C(=O)-NH-, -C(=O)-NR^{a}-, -O-C(=O)-NR^{a}-, and-C(=O)-NR^{a}R^{b}, wherein R^{a} and R^{b} are as defined above, wherein the aliphatic group optionally comprises at least one halogen atom,
      and
   - an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

Although Formulas (Ii) and (Ij) only show one isomer with respect to the constitution at the double bond this does not mean that only this isomer is meant but rather displays one isomer as representative for all isomers. Hence the cis- as well as the trans- isomers as well as mixtures of the two are included in the general description of the compounds and structures.

In some embodiments, one of R³² and R³³ in Formulas (Ii) and (Ij) is a hydrogen and the other of R³² and R³³ is an aliphatic group having a carbon number of from 1 to 18 in which one or more hydrogen atoms may be substituted by a halogen atom. Preferred examples include methyl, ethyl, propyl, isopropyl, butyl, isobutyl, sec-butyl, tert-butyl, n-pentyl, n-hexyl, 2-ethylhexyl, n-heptyl, n-octyl, n-nonyl, and n-decyl. In this embodiment, R⁷ in Formulas (Ii) and (Ij) is preferably an aliphatic group having a carbon number of from 1 to 18 in which one or more hydrogen atoms may be substituted by a halogen atom. Preferably, either R³² or R³³ is an aliphatic group having a carbon number of from 1 to 6 and, more preferably, an aliphatic group having a carbon number of from 1 to 4, which is substituted by at least one fluorine atom. Examples of such acid generating compound (ii) include those in Table 17:

**Table 17**

| | |
|---|---|
| 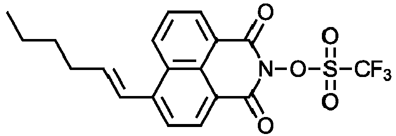 | D-6 |
| 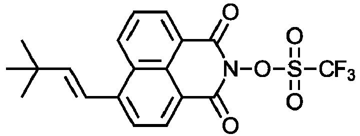 | D-7 |

In other embodiments, one of R³² and R³³ in Formulas (Ii) and (Ij) is a hydrogen and the other of R³² and R³³ is an aliphatic group having a carbon number of from 2 to 18 which comprises at least one -C(=O)-O- moiety. In an embodiment, one of R³² and R³³ in Formulas (Ii) and (Ij) is an aliphatic group having a carbon number of from 2 to 18 which comprises at least one -C(=O)-O- moiety and R⁷ is an aliphatic group having a carbon number of from 1 to 18, which may be substituted by one or more halogen atom(s). In this embodiment, preferably R⁷ is an aliphatic group having a carbon number of from 1 to 6 and, more preferably, an aliphatic group having a carbon number of from 1 to 4, which is substituted by at least one fluorine atom. Examples of such acid generating compounds (ii) include those in Table 18:

**Table 18**

| | |
|---|---|
| 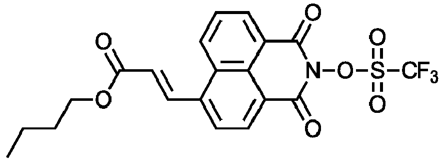 | D-1 |
| 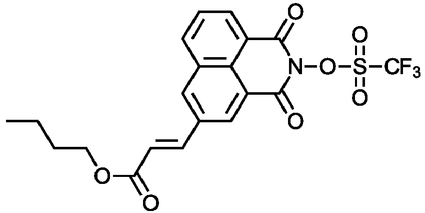 | D-11 |
| 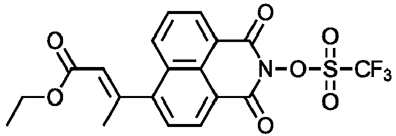 | D-18 |
| 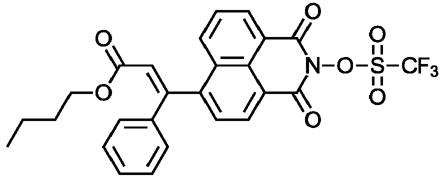 | D-19 |
| 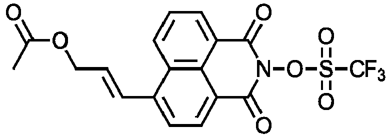 | D-21 |
| 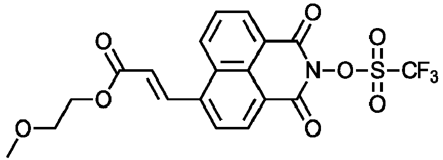 | D-23 |

In yet other embodiments, one of R³² and R³³ in Formulas (Ii) and (Ij) is a hydrogen and the other of R³² and R³³ is an aryl or heteroaryl group having a carbon number of from 4 to 18 in which one or more hydrogen atoms may be substituted by an aliphatic group or an alkoxy group and R⁷ is an aliphatic group having a carbon number of from 1 to 18, which may be substituted by one or more halogen atom(s). In such embodiments where the aryl or heteroaryl group is substituted by an aliphatic group or an alkoxy group, preferably the aliphatic group (and the aliphatic portion of the alkoxy group) has a carbon number of from 1 to 6 and, more preferably, a carbon number of from 1 to 4. In this embodiment, preferably R⁷ is an aliphatic group having a carbon number of from 1 to 6 and, more preferably, an aliphatic group having a carbon number of from 1 to 4, which is substituted by at least one fluorine atom. Examples of such acid generating compounds (ii) include those in Table 19:

**Table 19**

| | |
|---|---|
| 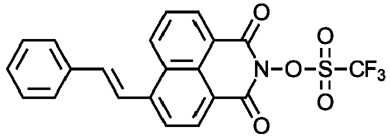 | D-2 |
| 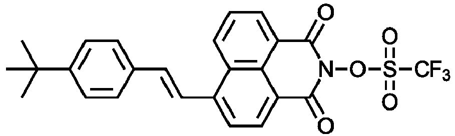 | D-3 |
| 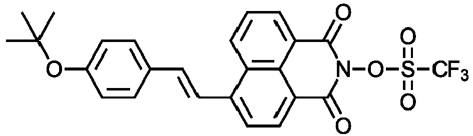 | D-4 |
| 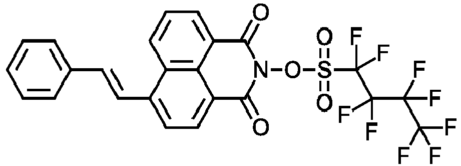 | D-5 |
| 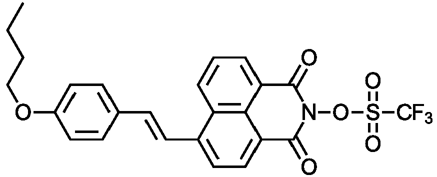 | D-9 |
| 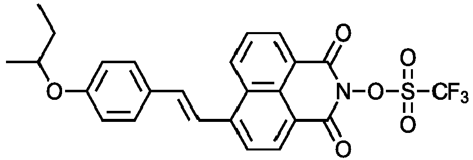 | D-10 |
| 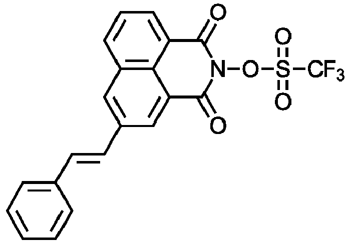 | D-12 |

In still other embodiments, R³² and R³³ in Formulas (Ii) and (Ij) are connected to from an alicyclic group. In some embodiments, the alicyclic group is a bicyclic group. In this embodiment, R⁷ in Formulas (Ii) and (Ij) is an aliphatic group having a carbon number of from 1 to 18 in which one or more hydrogen atoms may be substituted by a halogen atom. Examples of such acid generating compounds (ii) include those in Table 20:

**Table 20**

| | |
|---|---|
| 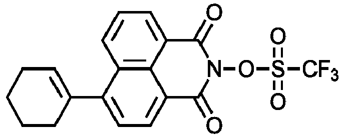 | D-16 |
| 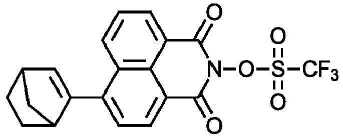 | D-17 |

In still other embodiments, one of R³² and R³³ in Formulas (Ii) and (Ij) is a hydrogen and the other of R³² and R³³ is a -C(=O)-NR^{a}- or a -C(=O)-NR^{a}R^{b}, wherein R^{a} and R^{b} are each independently an aliphatic group with a carbon number of from 1 to 10, which may be the same or different and may be connected to form an alicyclic group. In this embodiment, R⁷ in Formulas (Ii) and (Ij) is an aliphatic group having a carbon number of from 1 to 18 in which one or more hydrogen atoms may be substituted by a halogen atom. Examples of such acid generating compounds (ii) include those in Table 21:

**Table 21**

| | |
|---|---|
| 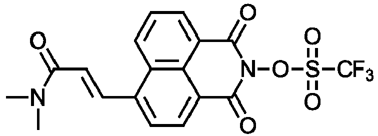 | D-8 |
| 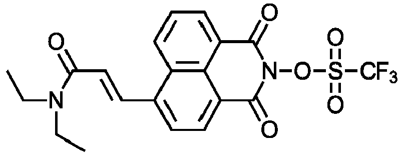 | D-14 |
| 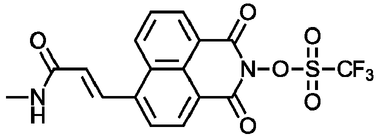 | D-15 |
| 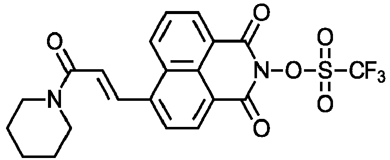 | D-20 |

In still other embodiments, one of R³² and R³³ in Formulas (Ii) and (Ij) is a hydrogen and the other of R³² and R³³ is a -CN or an aliphatic group having a carbon number of from 1 to 18 which comprises at least one -O-. In this embodiment, R⁷ in Formulas (Ii) and (Ij) is an aliphatic group having a carbon number of from 2 to 18 in which one or more hydrogen atoms may be substituted by a halogen atom. Examples of such acid generating compounds (ii) include those in Table 22:

**Table 22**

| | |
|---|---|
| 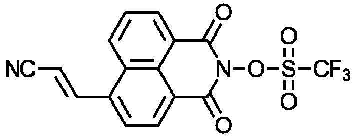 | D-13 |
| 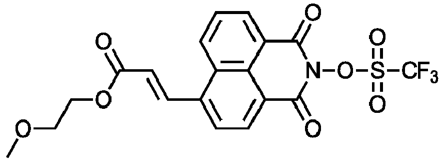 | D-22 |

In the most preferred embodiments of the compounds of Formulas (Ii) and (Ij) according to the present disclosure, R⁷ is -CF₃.

There is no particular limitation for the method for producing the N-hydroxynaphthalimide sulfonate derivative compounds of the present disclosure, and any known synthesis can be used to make the compounds of Formulas (Ii) and (Ij). Two exemplary routes are illustrated in Scheme 1 below. Compounds with a triple-bond substituent at 3 position can be similarly synthesized by starting from 3-bromo anhydride. The starting anhydrides (4-bromo-1,8-naphthalic anhydride and 3-bromo-1,8-naphthalic anhydride) are commercially available.

As shown in Scheme 5, Heck coupling between 4-bromo-1,8-naphthalic anhydride and an alkene affords naphthalic anhydrides with a double-bond group. Note that only E-isomer of the alkene is shown in Scheme 5 for clarity. These anhydrides are converted to the final N-hydroxynaphthalimide sulfonate derivatives via two different approaches. For all naphthalic compound containing a double-bond group, only one of the Z- or E-isomers is shown in Scheme 5. The practical experiment may generate Z-isomer, E-isomer, or a mixture of Z- and E-isomer in various ratio for the double-bond substituents. The first approach involves an one-pot reaction using 2.2 equivalents of triflic anhydride. The second approach allows for the isolation of N-hydroxyl imide intermediates and only 1.25 equivalent of triflic anhydride is required.

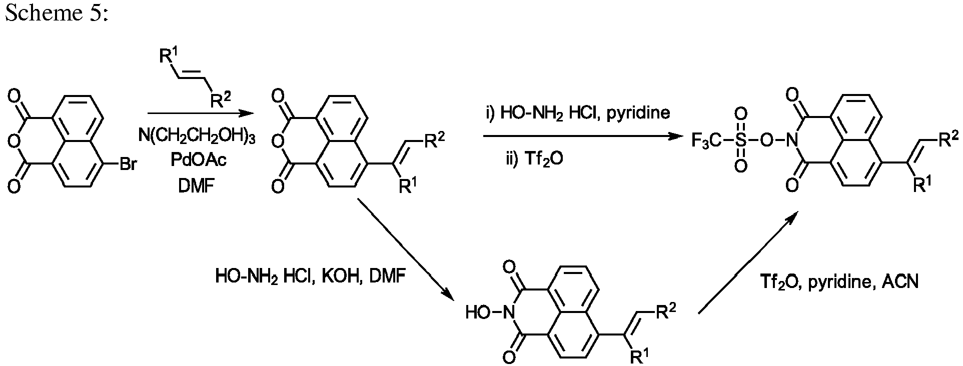

In addition to the acid generating compound (ii) according to the first, the second, the third and the fourth embodiment as described above also suitable as acid generating compounds (ii) are those compounds
- that are defined in claim 1 of WO 2014/084269 A1,
- that are defined in claim 1 of WO 2014/073409 A1,
- that are defined in claim 1 of WO 2015/046501 A1,
- that are defined in claim 1 of US 8,680,268 B2, or
- that have the general structure according to *"formula (A)"* as defined in claim 7 of US 8, 722,318 B2,

### Further components in the composition

The composition according to the present invention preferably further comprises
(iii) at least one compound capable of being imparted with an altered solubility in an aqueous solution in the presence of an acid;
(iv) at least one organic solvent;
   and
(v) optionally at least one additive being different from components (i), (ii), (iii) and (iv).

The composition according to the present invention can be used as a photoresist in a variety of applications, in particular for the production of electronic devices, including flat panel display (in this case the photoresist can be coated glass substrate or a layer of indium tin oxide) and a semiconductor device (in this case the photoresist can be coated onto a silicon wafer substrate). Various exposure radiations can be used, including an exposure with electromagnetic radiation having a wavelength in the range of 200 to 500 nm, more particularly in the range from 300 to 450 nm, more particularly in the range from 350 to 440 nm and even more particularly electromagnetic radiation having a wavelength of 365 nm (i-line), 435 nm (g-line) or 405 nm (h-line), wherein an electromagnetic radiation with a wavelength of 365 nm is most preferred.

The composition according to the present invention may comprise as component (iii) one or more photoresist polymers or copolymers, which may be base soluble or insoluble. The photoresist composition according to the present invention may be a positive tone or negative tone composition. In the case of a positive tone composition the solubility of component (iii) is increased upon reaction with the acid released from the compound according to the present invention. In this case, photoresist polymers or copolymers with acid labile groups are used as component (iii) which are insoluble in aqueous base solution, but which in the presence of the acid are catalytically deprotected such that they become soluble in an aqueous base solution. In the case of a negative tone composition, the solubility of component (iii) is decreased upon reaction with the acid released from the compound according to the present invention. In this case, photoresist polymers or copolymers are used as component (iii) which are soluble in aqueous base solution), but which in the presence of the acid are crosslinked such that they become insoluble in an aqueous base solution.

Examples of photoresist polymers that may be used as component (iii) in a positive tone composition include without limitation, aromatic polymers, such as homopolymers or copolymers of hydroxystyrene protected with an acid labile group; acrylates, such as for example, poly(meth)acrylates with at least one unit containing a pendant alicyclic group, and with the acid labile group being pendant from the polymer backbone and/or from the aclicyclic group, cycloolefin polymers, cycloolefin maleic anhydride copolymers, cycloolefin vinyl ether copolymers, siloxanes; silsesquioxanes, carbosilanes; and oligomers, including polyhedral oligomeric silsesquioxanes, carbohydrates, and other cage compounds. The foregoing polymers or oligomers are appropriately functionalized with aqueous base soluble groups, acid-labile groups, polar functionalities, and silicon containing groups as needed.

Examples of copolymers that may be used as component (iii) in the positive tone compositions of the present invention include without limitation poly(p-hydroxystyrene)-methyl adamantyl methacrylate (PHS-MAdMA), poly(p-hydroxystyrene)-2-ethyl-2-adamantyl methacrylate (PHS-EAdMA), poly(p-hydroxystyrene)-2-ethyl-2-cyclopentyl methacrylate (PHS-ECpMA), poly(p-hydroxy-styrene)-2-methyl-2-cyclopentyl methacrylate (PHS-MCpMA) or PHS-EVE.

Preferably, the at least one component (iii) in a positive tone composition is a poly(hydroxystyrene)-resin in which at least a part of the hydroxy groups is substituted by protective groups. Preferred protective groups are selected from the group consisting of a tert-butoxycarbonyloxy group, a tert-butyloxy group, a tert-amyloxycarbonyloxy group and an acetal group. Furthermore suitable as component (iii) are all the polymers and copolymers which in paragraphs [0068] to [0114] of EP 1 586 570 A1 are described as *"acid-dissociable group-containing resin".* The disclosure of EP 1 586 570 A1 with respect to these resins is enclosed herein by reference a forms a part of the disclosure of the present invention.

Preferred negative tone compositions comprise a mixture of materials that will cure, crosslink or harden upon exposure to acid. Preferred negative acting compositions comprise, as component (iii), a polymer binder such as a phenolic or non-aromatic polymer, a crosslinker component as an additive (v), the sensitizer compound (i) and the acid generating compound (ii). Suitable polymer binders and crosslinkers for such negative tone photoresist compostions and the use thereof have been disclosed in EP-A-0 164 248 and US 5,128,232. Preferred phenolic polymers for use as component (iii) include novolaks and poly(vinylphenol)s. Novolak resins are the thermoplastic condensation products of a phenol and an aldehyde. Examples of suitable phenols for condensation with an aldehyde, especially formaldehyde, for the formation of novolak resins include phenol, m-cresol, o-cresol, p-cresol, 2,4-xylenol, 2,5-xylenol, 3,4-xylenol, 3,5-xylenol and thymol. An acid catalyzed condensation reaction results in the formation of a suitable novolak resin which may vary in molecular weight from about 500 to 100,000 daltons. Polyvinyl phenol resins are thermoplastic polymers that may be formed by block polymelization, emulsion polymerization or solution polymerization of the corresponding monomers in the presence of a cationic catalyst. Vinylphenols useful for the production of polyvinyl phenol resins may be prepared, for example, by hydrolysis of commercially available coumarin or substituted coumarins, followed by decarboxylation of the resulting hydroxy cinnamic acids. Useful vinylphenols may also be prepared by dehydration of the corresponding hydroxy alkyl phenols or by decarboxylation of hydroxy cinnamic acids resulting from the reaction of substituted or nonsubstituted hydroxybenzaldehydes with malonic acid. Preferred polyvinyl phenol resins prepared from such vinylphenols have a molecular weight range of from about 2,000 to about 60,000 daltons. Preferred crosslinkers for use as component (v) include amine-based materials, including melamine, glycolurils, benzoguanamine-based materials and urea-based materials. Melamine-formaldehyde polymers are often particularly suitable. Such crosslinkers are commercially available, e.g. the melamine polymers, glycoluril polymers, urea-based polymer and benzoguanamine polymers, such as those sold by Cytec under tradenames Cymel 301, 303, 1170, 1171, 1172, 1123 and 1125 and Beetle 60, 65 and 80.

As component (iv) the composition according to the present invention comprises at least one organic solvent.

The organic solvent may be any solvent capable of dissolving components (i), (ii) and (iii) to generate a uniform solution, and one or more solvents selected from known materials used as the solvents for conventional chemically amplified resists can be used. Specific examples of the organic solvent include ketones such as acetone, methyl ethyl ketone, cyclohexanone, methyl isoamyl ketone and 2-heptanone, polyhydric alcohols and derivatives thereof such as ethylene glycol, ethylene glycol monoacetate, diethylene glycol, diethylene glycol monoacetate, propylene glycol, propylene glycol monoacetate, dipropylene glycol, or the monomethyl ether, monoethyl ether, monopropyl ether, monobutyl ether or monophenyl ether of dipropylene glycol monoacetate, cyclic ethers such as dioxane, and esters such as methyl lactate, ethyl lactate (EL), methyl acetate, ethyl acetate, butyl acetate, methyl pyruvate, ethyl pyruvate, methyl methoxypropionate, and ethyl ethoxypropionate. These organic solvents can be used alone, or as a mixed solvent containing two or more different solvents. Particularly preferred organic solvents (iv) are selected from the group consisting of a ketone, an ether and ester, wherein the use of PGMEA as the solvent.

Furthermore, the composition according to the present invention may also comprise at least one additive being different from components (i), (ii), (iii) and (iv). Suitable additives include surfactants for improving the ease of application, dissolution inhibitors, plasticizers, stabilizers, colorants, halation prevention agents and - in case of negative tone compositions - the above mentioned crosslinkers.

One additive (v) that is typically employed in those cases in which the composition according to the present invention comprises an acid generating compound (ii) according to the first or the second particular embodiment (i. e. an acid generating compound (ii) compound of the general structure (Ia), (Ib), (Ic) or (Id)) is a basic quencher. The basic quencher is for purposes of neutralizing acid generated in the surface region of the underlying photoresist layer by stray light which reaches what are intended to be unexposed (dark) regions of the photoresist layer. This allows for improvement in depth of focus in the defocus area and exposure latitude by controlling unwanted deprotection reaction in the unexposed areas. As a result, irregularities in the profile, for example, necking and T-topping, in formed resist patterns can be minimized or avoided.

To allow for effective interaction between the basic quencher and the acid generated in the dark areas of the underlying photoresist layer, the basic quencher should be of a non-surfactant-type. That is, the basic quencher should not be of a type that migrates to the top surface of the overcoat layer due, for example, to a low surface free energy relative to other components of the overcoat composition. In such a case, the basic quencher would not be appreciably at the photoresist layer interface for interaction with the generated acid to prevent acid deprotection. The basic quencher should therefore be of a type that is present at the overcoat layer/photoresist layer interface, whether being uniformly dispersed through the overcoat layer or forming a graded or segregated layer at the interface. Such a segregated layer can be achieved by selection of a basic quencher having a high surface free energy relative to other components of the overcoat composition.

Suitable basic quenchers include, for example: linear and cyclic amides and derivatives thereof such as N,N-bis(2-hydroxyethyl)pivalamide, N,N-diethylacetamide, N1,N1,N3,N3-tetrabutylmalonamide, 1-methylazepan-2-one, 1-allylazepan-2-one and tert-butyl 1,3-dihydroxy-2-(hydroxymethyl)propan-2-ylcarbamate; aromatic amines such as pyridine, and di-tert-butyl pyridine; aliphatic amines such as triisopropanolamine, n-tert-butyldiethanolamine, tris(2-acetoxy-ethyl)amine, 2,2',2",2'"-(ethane-1,,2-diylbis(azanetriyl))tetraethanol, and 2-(dibutylamino)ethanol, 2,2',2"-nitrilotriethanol; cyclic aliphatic amines such as 1-(tert-butoxycarbonyl)-4-hydroxypiperidine, tert-butyl-1-pyrrolidinecarboxylate, tert-butyl-2-ethyl-1H-imidazole-1-carboxylate, di-tert-butylpiperazine-1,4-dicarboxylate and N(2-acetoxy-ethyl)-morpholine. Of these basic quenchers, 1-(tert-butoxycarbonyl)-4-hydroxypiperidine and triisopropanolamine are preferred. While the content of the basic quencher will depend, for example, on the content of the photoacid generator in the underlying photoresist layer, it is typically present in an amount of from 0.1 to 5 wt %, preferably from 0.5 to 3 wt %, more preferably from 1 to 3 wt %, based on total solids of the overcoat composition.

According to a particularly preferred embodiment of the composition according to the present invention the composition comprises
(i) 0.01 to 40 wt.-%, preferably 0.01 to 10.0 wt.-% and more preferably 0.02 to 5.0 wt.-% of the sensitizer compound;
(ii) 0.01 to 10 wt.-%, preferably 0.01 to 5.0 wt.-% and more preferably 0.02 to 4.0 wt.-% of the acid generating compound;
(iii) 5 to 90 wt.-%, preferably 5 to 30 wt.-% and more preferably 5 to 20 wt.-% of the compound capable of being imparted with an altered solubility in an aqueous solution in the presence of an acid component;
(v) 0 to 10 wt.-%, preferably 0.001 to 1.0 wt.-% and more preferably 0.01 to 0.1 wt.-% of the further additive;
wherein the reminder in the composition is the organic solvent (iv).

As in the case of using an acid generating compound (ii) according to the third particular embodiment compounds according to the present invention (i. e. an acid generating compound (ii) of the general structure (Ie), (If), (Ig) or (Ih)) the functional basic group serving as a quencher for the acid group that is released upon exposure to electromagnetic radiation is a part of the photoacid generator compound, it is not necessary to add a separate basic component as a quencher (as it is necessary in the photoresist compositions known from the prior art). According to a preferred embodiment of the composition according to the present invention this composition comprises an acid generating compound (ii) having the above described general structure (Ie), (If), (Ig) or (Ih) and comprises less than 5 wt.-%, more preferably less than 1 wt.-%, even more preferably less than 0.1 wt.% and most preferably 0 wt.-% of a basic compound being different from components (i) through (v), such as hydroxides, carboxylates, amines, imines, and amides.

A contribution towards solving the objects of the present invention is also made by a process for producing a composite comprising a substrate and a coating that is applied onto the substrate in a patterned structure, the process comprising the steps of
(α1) applying a layer of the composition according to the present invention, preferably the composition that, besides components (i) and (ii), further comprises components (iii), (iv) and optionally (v), onto the surface of the substrate and at least partial removal of the organic solvent (iv);
(α2) exposing selected areas of the layer to electromagnetic radiation, thereby releasing an acid from the compound (ii) in the areas exposed to the electromagnetic radiation;
(α3) optionally heating the layer to impart compound (iii) in the areas in which the acid has been released with an altered solubility in an aqueous solution;
(α4) optionally at least partial removal of the layer.

In process step (α1) a layer of the composition according to the present invention, preferably a layer of the composition that, besides components (i) and (ii), further comprises components (iii), (iv) and optionally (v), is applied onto the surface of the substrate and the organic solvent (iv) is at least partially removed.

Substrates may be any dimension and shape, and are preferably those useful for photolithography, such as silicon, silicon dioxide, silicon-on-insulator (SOI), strained silicon, gallium arsenide, coated substrates including those coated with silicon nitride, silicon oxynitride, titanium nitride, tantalum nitride, ultrathin gate oxides such as hafnium oxide, metal or metal coated substrates including those coated with titanium, tantalum, copper, aluminum, tungsten, alloys thereof, and combinations thereof. Preferably, the surfaces of substrates herein include critical dimension layers to be patterned including, for example, one or more gate-level layers or other critical dimension layer on the substrates for semiconductor manufacture. Such substrates may preferably include silicon, SOI, strained silicon, and other such substrate materials, formed as circular wafers having dimensions such as, for example, 20 cm, 30 cm, or larger in diameter, or other dimensions useful for wafer fabrication production.

Application of the composition according to the present invention onto the substrate may be accomplished by any suitable method, including spin coating, spray coating, dip coating, doctor blading, or the like. Applying the layer of photoresist is preferably accomplished by spin-coating the photoresist using a coating track, in which the photoresist is dispensed on a spinning wafer. During dispense, the wafer may be spun at a speed of up to 4,000 rpm, preferably from about 500 to 3,000 rpm, and more preferably 1,000 to 2,500 rpm. The coated wafer is spun to remove the organic solvent (iv), and baked on a hot plate to remove residual solvent and free volume from the film to make it uniformly dense.

In process step (α2) selected areas of the layer are exposed to electromagnetic radiation, thereby releasing an acid from the compound (ii) in the areas exposed to the electromagnetic radiation. As stated above, various exposure radiations can be used, including an exposure with electromagnetic radiation having a wavelength of 365 nm (i-line), 435 nm (g-line) or 405 nm (h-line), wherein electromagnetic radiation having a wavelength of 365 nm is particularly preferred.

Such a pattern-wise exposure can be carried out using an exposure tool such as a stepper, in which the film is irradiated through a pattern mask and thereby is exposed pattern-wise. The method preferably uses advanced exposure tools generating activating radiation at wavelengths capable of high resolution including extreme-ultraviolet (EUV) or e-beam radiation. It will be appreciated that exposure using the activating radiation decomposes compound (ii) that is contained in the photoresist layer in the exposed areas and generates acid and decomposition byproducts, and that the acid then effects a chemical change in the polymer compound (iii) (deblocking the acid sensitive group to generate a base-soluble group, or alternatively, catalyzing a cross-linking reaction in the exposed areas). The resolution of such exposure tools may be less than 30 nm.

In process step (α3) the layer can optionally be heated to impart compound (iii) in the areas in which the acid has been released with an altered solubility in an aqueous solution. In this so called "*post*-*exposure bake"* the solubility differences between exposed and unexposed regions of the coating layer are created or enhanced. Typically post-exposure bake conditions include temperatures of about 50°C or greater, more specifically a temperature in the range of from about 50°C to about 160°C for 10 seconds to 30 minutes, preferably for 30 to 200 seconds. According to a particular embodiment of the process according to the present invention no heat treatment is performed after process step (α2) and before (α4).

In process step (α4) the layer can at least partially be removed with an aqueous solution, preferably an aqueous base solution. This can be accomplished by treating the exposed photoresist layer with a suitable developer capable of selectively removing the exposed portions of the film (where the photoresist is positive tone) or removing the unexposed portions of the film (where the photoresist is negative tone). Preferably, the photoresist is positive tone based on a polymer having acid sensitive (deprotectable) groups, and the developer is preferably a metal-ion free tetraalkylammonium hydroxide solution.

A contribution towards solving the objects of the present invention is also made by a composite obtained by the process according to the present invention. This composite is characterised in that it comprises a substrate and a coating applied on the surface of the substrate in a patterned structure, wherein the coating comprises a compound according to the present invention.

A contribution towards solving the objects of the present invention is also made by a composite comprising a substrate and a coating applied on the surface of the substrate in a patterned or non-patterned structure, wherein the coating comprises
- a sensitizer compound (i) as defined above
   and
- an acid generating compound (ii) as defined above or the fragment thereof that remains if in general structure (I) the acid group is cleaved.

For example, that fragment of the acid generating compound (ii) that remains if in general structure (I) the acid group is cleaved may have the following general structure: 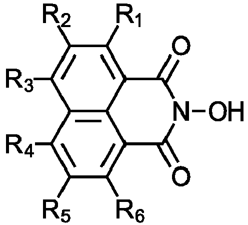 wherein R¹, R², R³, R⁴, R⁵ and R⁶ are as defined above.

A contribution towards solving the objects of the present invention is also made by the use a combination of a sensitizer compound (i) and an acid generating compound (ii) as defined above for photoinduced polymerization, photoinduced crosslinking, photoinduced degradation, photoinduced deprotection, photoinduced color change, photoinduced transformation of functional groups or any combination of at least two of them. This combination of a sensitizer compound (i) and an acid generating compound (ii) is in particular suitable for use in in protective coatings, printing plates, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufacturing.

The invention is now explained in more detail with the aid of non-limiting examples.

### EXAMPLES

All the compounds and the solvents were of experimental grade. ¹H, ¹³C and ¹⁹F-NMR spectra were measured on a Bruker AV-400 spectrometer with chemical shifts reported as ppm (in CDCl₃/DMSO-d6, TMS as internal standard). Mass spectra were measured on a Shimadzu GCMS-QP2010SE spectrometer. Melting points were determined by an Electrothermal-melting point apparatus and are uncorrected. UV-vis. absorption spectra were determined on a DU 800 UV-vis spectrometer.

### Example 1: Synthesis of compound S-3:

### Synthesis of compound A3:

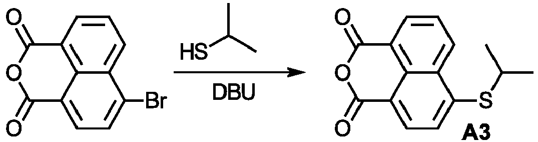

In a 3 L flask was charged 4-bromo-1,8-naphthalic anhydride (230 g, 0.83 mol), 1 L of DMAc, and 2-propylthiol (68.3 g, 0.897 mol). DBU (137.7 g, 0.897 mol) was added dropwise to this slurry mixture, and the temperature was kept under 60 °C. After the addition was complete, the reaction mixture was stirred at 55 °C overnight. The reaction mixture was cooled to room temperature, and 1 L of a 1 : 1 mixture of DI water and MeOH was then added. The mixture was filtered to give a yellow solid which was dried under vacuum at 50 °C overnight to afford 212 g of the anhydride A3 (yield: 93%). Mp: 122-9 °C. Note that A3 was used in the subsequent reaction without further purification.

### Synthesis of compound H3:

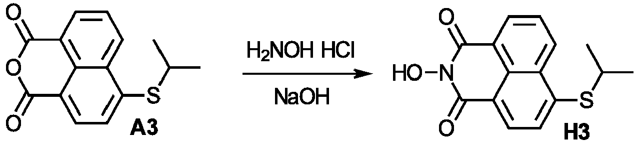

To a 5 L flask was charged A3 (212 g, 0.78 mol), 1 L of DMAc, and H₂NOH·HCl (57.0 g, 0.82 mol). To the slurry mixture was added dropwise 48% NaOH solution (32.8 g, 0.82 mol), and the temperature was kept under 25 °C during the addition. After the addition was complete, the reaction mixture was stirred at room temperature overnight. The mixture was then heated to 80 °C and kept at the same temperature for 3 h. The reaction mixture was cooled to room temperature, and 1 L of a 1 : 3 mixture of MeOH and DI water was then added. The mixture was stirred at room temperature for 2 h. Filtration gave the solid which was washed with 100 mL of MeOH and 100 mL of CH₂Cl₂. The yellow solid was dried under vacuum at 50 °C overnight to afford 178 g of the hydroxyimide H3 (yield: 80%). Mp: 179-182°C. Note that H3 was used in the subsequent reaction without further purification.

### Synthesis of compound S-3:

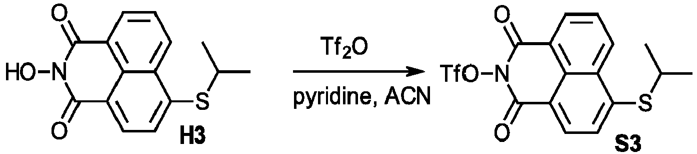

To a 2 L flask was charged H3 (142 g, 0.495 mol), acetonitrile (830 g) and pyridine (117.5 g, 1.48 mol). The mixture was cooled to 0 °C, and triflic anhydride (188.5 g, 0.669 mol) was then added dropwise below 5 °C. After the addition, the reaction mixture was allowed to warm to room temperature and stirred overnight. The reaction mixture was heated to make all solids dissolved and then cooled to room temperature. 1 L of 1.0 M HCl solution was then added. The mixture was stirred at room temperature for 30 min. Filtration gave the yellow solid. The solid was dissolved in 600 g of CH₂Cl₂, and the solution was passed through a pad of silica gel. Removal of CH₂Cl₂ and recrystallization from 300 g of a 1 : 1 mixture of isopropanol and acetonitrile gave a yellow solid which was dried under vacuum at 50 °C overnight to afford 154.5 g of S-3 (yield: 74%). Mp: 125.5-126 °C. ¹H NMR (300 MHz, CDCl₃) δ: 8.62 (dd, 1H), 8.58 (dd, 1H), 8.44 (d, 1H), 7.71 (t, 1H), 7.57 (d, 1H), 3.70 (septet, 1H), 1.42 (d, 6H).

### Example 2: Synthesis of compound T41:

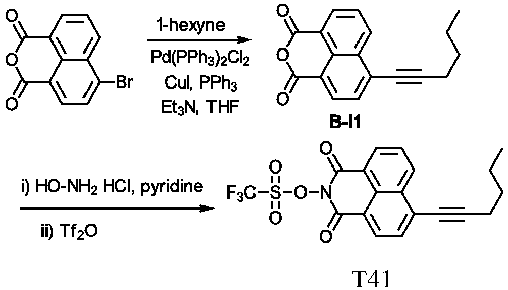

To a 5 L flask was charged 4-bromo-1,8-naphthalic anhydride (263 g, 0.9492 mol), PPh₃ (19.92 g, 75.94 mmol), Et₃N (201.7 g, 1.99 mol) and 2 L of THF. The mixture was stirred under nitrogen for 1 h. To this mixture was added CuI (5.42 g, 28.5 mmol) and Pd(PPh₃)₂Cl₂ (6.66 g, 9.492 mmol) under nitrogen. The mixture was heated to reflux, and 1-hexyne (101.8 g, 1.0 mol) in 0.5 L of THF was added dropwise in 2.5 h. After the addition, the mixture was kept at reflux for 14.5 h. TLC monitoring of the reaction showed a small amount of the unreacted anhydride. An additional amount of 1-hexyne (14.5 g, 142 mmol) was added in 0.5 h, and the mixture was kept at reflux for an additional 2 h. The mixture was allowed to slowly cool down to room temperature. 20 mL of DI water was added. Filtration gave a yellow solid, and the complete removal of solvents from the filtrate gave a dark brown solid. Both solids were combined and then dissolved in 1 L of CH₂Cl₂. The solution was washed with 1.5 L of DI water. Separation and rotavap of the CH₂Cl₂ solution gave 208 g of the crude solid. Recrystallization from 320 mL of ACN gave 200 g (yield: 70%) of T41 as light yellow crystals. Mp: 153-4 °C. ¹H NMR (300 MHz, CDCl₃) δ: 8.50 (d, 2H), 8.25 (d, 1H), 7.65 (t, 1H), 7.58 (d, 1H), 2.53 (t, 2H), 1.62 (p, 2H), 1.45 (sextet, 2H), 0.91 (t, 3H). ¹³C NMR (75.5 MHz, CDCl₃) δ: 13.6, 19.6, 22.2, 30.5, 103.5, 117.0, 118.8, 127.5, 129.8, 130.2, 130.6, 131.7, 132.4, 133.4, 133.8, 160.0, 160.4.

### Example 3: Synthesis of compound Vd:

### Preparation of 6-Piperidino-2-oxa-1,3-phenalenedione

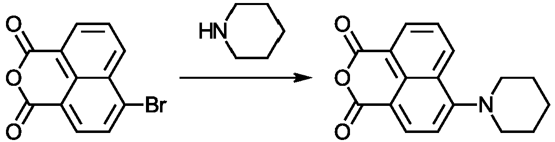

A mixture of 4-bromo-1,8-naphthalic anhydride (2.77 g, 10.0 mmol), and piperidine (1.97 ml, 20 mmol) in 2-methoxyethanol (5 ml) was refluxed for 16 h, then the reaction mixture was cooled to room temp to obtain a yellow solid, which was purified by recrystallization from ethanol to give 2.41 g (86%) of 6-Piperidino-2-oxa-1,3-phenalenedione in the form of orange needles. CAS: 87223-12-9. M.p. 170-172°C (lit. 175-176°C) ¹H NMR (400 MHz, CDCl₃) δ: 8.54 (d, 1H), 8.46 (d, 1H), 8.41 (d, 1H), 7.68 (t, 1H), 7.17 (d, 1H), 3.29 (s, 4H), 1.90 (s, 4H), 1.75 (s, 2H); ¹³C NMR (100 MHz, CDCl₃) δ: 161.5, 160.7, 158.4, 135.0, 133.1, 132.4, 132.2, 126.1, 125.6, 119.2, 114.9, 110.7, 54.3, 26.1, 24.2; MS m/z: 281(M+, 83), 280 ([M-1]+, 100).

### Preparation of 2-Hydroxy-6-piperidino-2-aza-2H-phenalene-1,3-dione

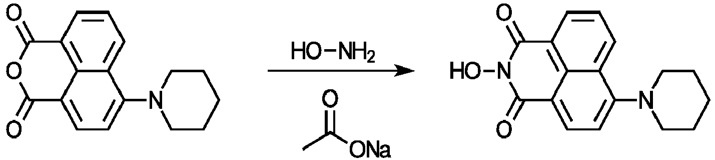

To a clean, dry 20 mL reaction vial equipped on magnetic stirring hot plate with thermosensor was added deionized water (10 mL), sodium acetate (0.3324 g, 4.0525 mmol), hydroxylamine hydrochloride (0.2915 g, 4.1947 mmol), and 4-piperidinyl-1,8-naphthalic anhydride (1 g, 3.5548 mmol). The reaction mixture was heated to 75-80°C and hold for 16.5 hrs. The reaction was then cooled to < 30°C and the mixture was filtered. The solids were slurred in deionized water (15 mL) and refiltered and washed with more deionized water. The solid was air-dried for 12 hours, and then washed with methanol (15 mL) twice. The solid was filtered and air-dried 12 hours, then dried in vacuo at 60°C for 24 hrs. 2.41 g (86 %) of the product obtained in the form of an orange powder. ¹H NMR (400 MHz, ppm, DMSO-d6) δ: 10.54 (s, 1H), 8.43 (d, 1H), 8.33 (d, 2H), 7.77 (t, 1H), 7.23 (t, 1H), 3.17 (s, 4H), 1.78 (s, 4H), 1.62 (s, 2H).

### Preparation of 6-Piperidino-2-(trifluoromethylsulfonyloxy)-2-aza-2H-phenalene-1,3-dione

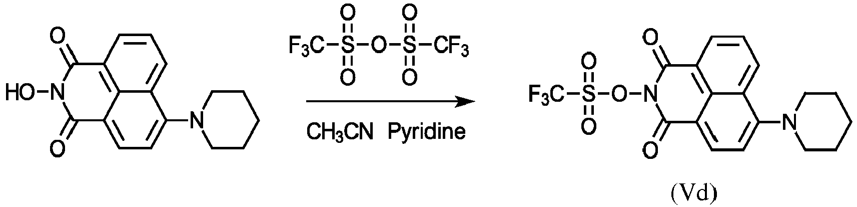

To a clean, dry 20 mL vial equipped with thermosensor, additional syringe, magnetic stirring, nitrogen balloon on cooling bath; was charged acetonitrile (10 mL), 2-Hydroxy-6-piperidino-2-aza-2H-phenalene-1,3-dione (1 g, 3.375 mmol), pyridine (0.41 mL, 5.062 mmol). The mixture was cooled to < 10°C and stirred. Triflic anhydride (0.6 mL, 5.062 mmol) was charged to the syringe and a slow addition was started. The reaction is held below 15°C during the addition. After the addition, the reaction is warmed to room temperature overnight. The reaction is then heated to 75-80°C until a clear solution is observed. The reaction is then cooled to < 10°C. The crude solid is then filtered washed with methanol and air-dried 12 hours, then dried in vacuo at 40°C for 48 hrs. The product is yellowish orange and was obtained in an amount of 0.90g (62.3 %). ¹H NMR (400 MHz, CDCl₃) δ: 8.60 (dd, 1H), 8.52 (d, 1H), 8.45 (dd, 1H), 7.72 (t, 1H), 7.20 (d, 1H), 3.29 (t, 4H), 1.90 (q, 4H), 1.75 (q, 2H), ¹⁹F NMR (400 MHz, CDCl₃) δ: -70.65 (s, CF3), UV-vis (nm) λmax=420 nm (0.001% in PGMEA)

### Example 4: Synthesis of compound D-2:

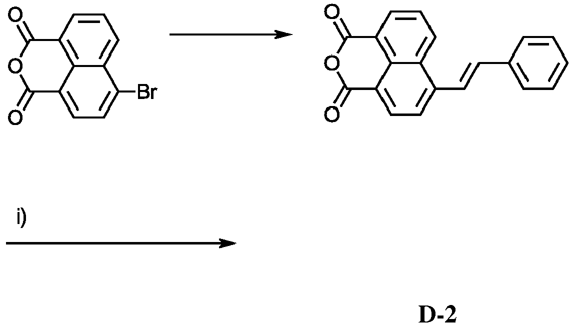

To a 1-L flask was charged 4-bromo-1,8-naphthalic anhydride (70 g, 0.253 mol), styrene (31.6 g, 0.303 mmol), triethanolamine (71.6 g, 0.48 mol), Pd(OAc)₂ (0.567 g, 0.00253 mol) and 70 g of DMF. The mixture was heated to 100-105 °C and stirred under nitrogen for 22 h. The mixture was allowed to slowly cool down to room temperature. 0.5 L of DI water was added. Filtration gave a solid which was dried under vacuum to afford 69 g (yield: 91%) of **D2-I** as light brown crystals. Note that **D2-I** was used in the subsequent reaction without further purification. Mp: 224-6°C.

To a 1 L flask was charged **D2-I** (69 g, 0.230 mol), H₂NOH·HCl (15.6 g, 0.241 mol), and pyridine (181.7 g, 2.30 mol). The mixture was heated to reflux for 4 h. TLC monitoring the reaction showed the disappearance of **D2-I.** The reaction mixture was cooled to 0°C using an ice-salt bath. To this mixture was added dropwise triflic anhydride (142.5 g, 0.506 mol), and the temperature was kept below 10°C during the addition. The addition was completed in 2.5 h. 2 L of DI water was added, and the resulting mixture stirred at room temperature for 1 h. Filtration gave 83 g of the yellow solid. Recrystallization from 1000 g of CH₂Cl₂ and 1000 g of MeOH gave 65 g (yield: 63%) of compound **D**-**2** as yellow powder. Mp: 182-4°C. ¹H NMR (300 MHz, DMSO) δ: 9.11 (d, 1H), 8.65 (d, 1H), 8.59 (d, 1H), 8.31 (d, 1H), 8.22 (d, 1H), 7.99 (dd, 1H), 7.85 (d, 2H), 7.65 (d, 1H), 7.45 (m, 3H).

### Example 5: Preparation of photoresist compositions

The following photoresist compositions have been prepared as follows: PHS-EVE resin polymer was totally dissolved in certain amount of PGMEA solvent and then the photoacid generating compound (i), a quencher and the sensitizer compound (ii) were added into the solution. The solution was shaken by an automatic shaker for at least 12 hours at room temperature. The solution was filtered through 1.0 µm Teflon filter.

### Composition A0 as reference

- 0.446 g **NIT** (N-trifluoromethylsulfonyl-oxy-1,8-naphthalimide) as component (ii)
- 15 g PHS-EVE (a tri-block copolymer obtained by living cationic polymerization of p-hydroxystyrene (PHS) and ethyl vinyl ether (EVE) with 30 % EVE) as component (iii)
- 85 g of PGMEA as solvent (component (iv))
- 0.026 g of triethylamine as quencher (component (v))

### Composition A1 as reference

- 0.223 g **2-ITX** (2-isopropylthioxanthone) as component (i)
- 0.446 g **NIT** as component (ii)
- 15 g PHS-EVE (30 % EVE) as component (iii)
- 85 g of PGMEA as component (iv)
- 0.026 g of triethylamine as component (v)

### Composition B0

- 0.550 g of compound **T41** as component (ii)
- 15 g PHS-EVE (30 % EVE) as component (iii)
- 85 g of PGMEA as component (iv)
- 0.013 g of triethylamine as component (v)

### Composition B1

- 0.223 g **2-ITX** as component (i)
- 0.549 g of compound **T41** as component (ii)
- 15 g PHS-EVE (30 % EVE) as component (iii)
- 85 g of PGMEA as component (iv)
- 0.013 g of triethylamine as component (v)

### Composition C0

- 0.542 g of compound **S**-**3** as component (ii)
- 15 g PHS-EVE (30 % EVE) as component (iii)
- 85 g of PGMEA as component (iv)
- 0.013 g of triethylamine as component (v)

### Composition C1

- 0.223 g **2-ITX** as component (i)
- 0.541 g of compound **S-3** as component (ii)
- 15 g PHS-EVE (30 % EVE) as component (iii)
- 85 g of PGMEA as component (iv)
- 0.013 g of triethylamine as component (v)

### Composition D0

- 0.542 g of compound **Vd** as component (ii)
- 15 g PHS-EVE (30 % EVE) as component (iii)
- 85 g of PGMEA as component (iv)
- 0.013 g of triethylamine as component (v)

### Composition D1

- 0.223 g **2-ITX** as component (i)
- 0.541 g of compound **Vd** as component (ii)
- 15 g PHS-EVE (30 % EVE) as component (iii)
- 85 g of PGMEA as component (iv)
- 0.013 g of triethylamine as component (v)

### Composition E0

- 0.542 g of compound **D-2** as component (ii)
- 15 g PHS-EVE (30 % EVE) as component (iii)
- 85 g of PGMEA as component (iv)
- 0.013 g of triethylamine as component (v)

### Composition E1

- 0.223 g **2-ITX** as component (i)
- 0.541 g of compound **D-2** as component (ii)
- 15 g PHS-EVE (30 % EVE) as component (iii)
- 85 g of PGMEA as component (iv)
- 0.013 g of triethylamine as component (v)

### Example 6: Preparation of patterned structures

The above described compositions are used to prepare a patterned structure by photolithography. Photo-patterning process was preceded with Coo1UV-100 Mask Aligner with i-line (365 nm) LED light source (4.0 mW/cm²). Proper exposure was determined by stepwise changing the exposure time (10 sec to 100 sec) and checking the pattern by a microscope which has the magnification of 500 times after development. Eop (optimum energy) of i-line exposure was determined by changing an exposure time and checking at least three 10 micron space patterns using a precise digital microscope under x 2800 ~ 1400 in magnification and 0.1 ~ 0.5 µm in resolution (KH-8700 model with MXG-10C as zoom lens and OL-700II as objective lens from Hirox Co., Japan). It is defined that Eop is the exposing energy where the 10 µm space pattern shows the same dimension than that of the mask.

In a first step, a coating of these compositions is applied on a pre-cleaned glass substrate (7.5 x 2.5 cm size) by means of a spin coater at 1000 rpm. The coated substrate is heated on a precision hotplate at 110°C for 60 sec and is subsequently exposed to electromagnetic radiation in the i-line aligner under each condition. The areas exposed to the radiation on the photoresist film is removed by means of a 2.38 wt.-% aqueous tetramethylammonium hydroxide (TMAH) solution. Residual TMAH and soluble PR on the patterned substrate were removed by dipping in deionized water for 30 seconds. The substrate was blew and dried by a nitrogen gas.

**Table 1:**

| **composition** | **Concentration of PAG (wt.-%)** | **Concentration of 2-ITX (wt.-%)** | **Eop (mJ/cm²)** | **factor Eop PAG/Eop Sens** |
|---|---|---|---|---|
| A0 | 0.45 | 0 | 196 | 1.23 |
| A1 | 0.45 | 0.22 | 160 | |
| B0 | 0.55 | 0 | 104 | 2.17 |
| B1 | 0.55 | 0.22 | 48 | |
| C0 | 0.54 | 0 | 120 | -- |
| C1 | 0.54 | 0.22 | n.d. | |
| D0 | 0.55 | 0 | 80 | 1.33 |
| D1 | 0.55 | 0.22 | 60 | |
| E0 | 0.58 | 0 | 400 | 2.50 |
| E1 | 0.58 | 0.22 | 160 | |

| | | | | |
|---|---|---|---|---|
| n. d. = not detected | | | | |

As can be seen, Eop for the compositions containing sensitizer is lower than that of the composition with PAG only. This means a shorter exposure time or lower energy is needed to change the solubility of the resist. Hence there is an increase in reactivity when a sensitizer is used and sensitized compositions can either be used to speed up production or reduce the content of PAG and sensitizer at the same production speed. I addition it becomes clear that the sensitizing factor (= Eop PAG/Eop Sens) is higher for compositions comprising a sensitizer compound.

## Claims

1. A composition comprising
(i) at least one sensitizer compound that, upon exposure to electromagnetic radiation, changes from a ground state into an excited state;
and
(ii) at least one acid generating compound that, upon transfer of energy or an electron from the sensitizer compound in its excited state to the acid generating compound, releases an acid, the acid generating compound having the general structure (I) 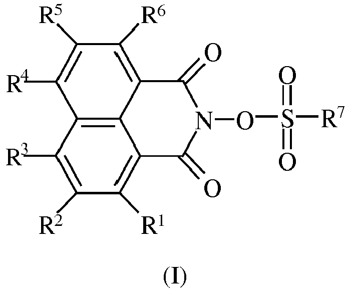 wherein
- R¹, R², R³, R⁴, R⁵ and R⁶ independently from each other represent a hydrogen atom or an organic residue, with the provisio that at least one of R¹, R², R³, R⁴, R⁵ and R⁶, preferably at least one of R² and R³, is an organic residue;
- R⁷ represents an organic residue.

2. The composition according to claim 1, wherein the sensitizer compound (i) is selected from the group consisting of xanthones, thioxanthones, coumarines, anthracenes, acridines, imidazoles, triazines, amino benzoates and a mixture of at least two thereof.

3. The composition according to claim 1 or 2, wherein the sensitizer compound (i) has the general structure (II) 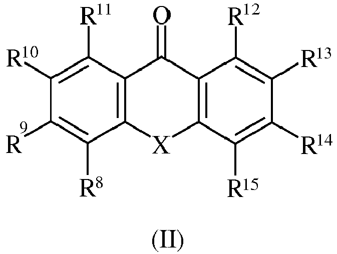 wherein R⁸, R⁹, R¹⁰, R¹¹, R¹², R¹³, R¹⁴ and R¹⁵ independently from each other represent a hydrogen atom, an organic residue, a hydroxy group, an amine group, a nitro group or a halogen, wherein two of these residues may form together an aliphatic or aromatic ring system, and X represents either O or S.

4. The composition according to anyone of claims 1 to 3, wherein the acid generating compound (ii) has the general structure (Ia) or (Ib) 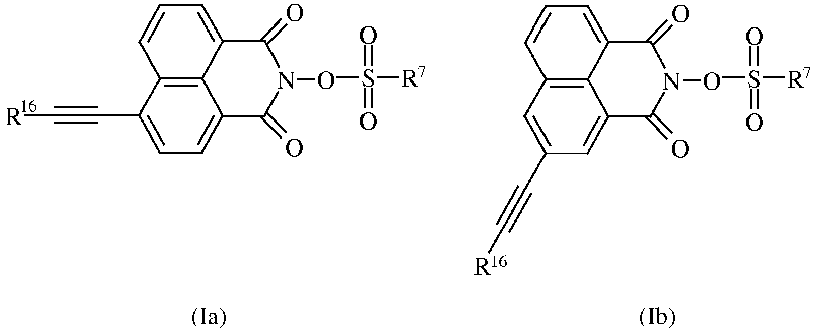 wherein
R¹⁶ is selected from the group consisting of
- a hydrogen atom,
- an aliphatic group having a carbon number of from 1 to 18 in which one or more hydrogen atoms may be substituted by a halogen atom,
and
- an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
R⁷ is selected from the group consisting of
- -CH₃, -CH₂F, -CHF₂, or -CF₃,
- an aliphatic group having a carbon number of from 2 to 18, which may be substituted by one or more halogen atom(s),
- an aliphatic group having a carbon number of from 2 to 18 which comprises at least one moiety selected from the group consisting of -O-, -S-, -C(=O)-, -C(=O)-O-,C(=O)-S-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
- an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
and
- an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

5. The composition according to anyone of claims 1 to 3, wherein the acid generating compound (ii) has the general structure (Ic) or (Id) 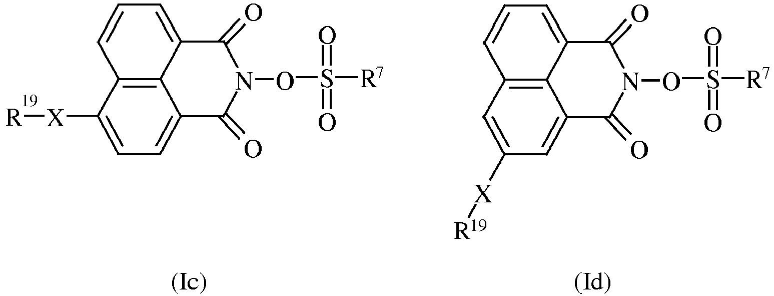 wherein
X is O or S;
R¹⁹ is selected from the group consisting of
- an aliphatic group having a carbon number of from 1 to 18 in which one or more hydrogen atoms may be substituted by a halogen atom,
- an aliphatic group having a carbon number of from 2 to 18 which comprises at least one moiety selected from the group consisting of -S-, -C(=O)-S-, -O-S(=O)₂-, -O-C(=O)-O-, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic group, and wherein the aliphatic group optionally comprises at least one halogen atom,
- a group represented by the Formula (C):
-R²⁰-Ar (C)
wherein
- R²⁰ is a single bond or an aliphatic group having a carbon number of from 1 to 20, which may comprise at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
and
- Ar is an aryl or heteroaryl group in which one or more hydrogen atoms may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
- a group represented by the Formula (D):
-R²¹-R²²-O-C(=O)-R²³-R²⁴ (D)
wherein
- R²¹ and R²² are each independently a aliphatic group having a carbon number of from 1 to 5,
- R²³ is an aliphatic group having a carbon number of from 1 to 10; and
- R²⁴ is an aliphatic group having a carbon number of from 1 to 18, which comprises at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, and wherein the at least one moiety, if more than one, may be separated by an aliphatic group,
and
- a group represented by the Formula (E):
-R²⁵-C(=O)-O-R²⁶ (E)
wherein
- R²⁵ is an aliphatic group having a carbon number of from 2 to 18, which may comprise at least one moiety selected from the group consisting of -O-, -S-, -C(=O)-S-, -O-S(=O)₂-, -O-C(=O)-O-, -C(=O)-NH-, -O-C(=O)-NH-, -C(=O)-NR^{a}-, and -O-C(=O)-NR^{a}-, wherein R^{a} is as defined above, and wherein the at least one moiety, if more than one, may be separated by a aliphatic group,
and
- R²⁶ is an aliphatic group having a carbon number of from 1 to 18 which comprises at least one moiety selected from the group consisting of -O-, -S-, -C(=O)-O-,-C(=O)-S-, -O-S(=O)₂-, -O-C(=O)-O-, -C(=O)-NH-, -O-C(=O)-NH-, -C(=O)-NRₐ-, -O-C(=O)-NR^{a}-, and -C(=O)-NR^{a}R^{b}, wherein R^{a} and R^{b} are as defined above, and wherein the at least one moiety, if more than one, may be separated by a aliphatic group,
R⁷ is selected from the group consisting of
- an aliphatic group having a carbon number of from 1 to 18, which may be substituted by one or more halogen atom(s),
- an aliphatic group having a carbon number of from 3 to 18 which comprises at least one moiety selected from the group consisting of -O-, -S-, -C(=O)-O-, -C(=O)-S-, -O-S(=O)₂-, -O-C(=O)-O-, -C(=O)-NH-, -O-C(=O)-NH-, -C(=O)-NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
- an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
and
- an arylalkyl or heteroarylalkyl group having a carbon number of from 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

6. The composition according to anyone of claims 1 to 3, wherein the acid generating compound (ii) has a general structure (I) in which at least one of R¹, R², R³, R⁴, R⁵ and R⁶ represents a group of the Formula (Fa) or (Fb)
-NR²⁷R²⁸ (Fa)
-A-NR²⁷R²⁸ (Fb)
wherein
R²⁷ and R²⁸ independently from each other represent a hydrogen atom, a linear or branched, optionally substituted aliphatic or heteroaliphatic group having 1-20 carbon atoms or an optionally substituted aromatic or heteroaromatic group having 6-20 carbon atoms, or
R²⁷ and R²⁸ together form a heterocyclic group that, in addition to the nitrogen atom shown in Formula (Fa) or (Fb), optionally comprises a further heteroatom selected from the group consisiting of N, O and S;
A represents an optionally substituted divalent aliphatic or heteroaliphatic group having 1-20 carbon atoms or a divalent aromatic or heteroaromatic group having 6-20 carbon atoms.

7. The composition according to anyone of claims 1 to 3, wherein the acid generating compound (ii) has the general structure (Ii) or (Ij) 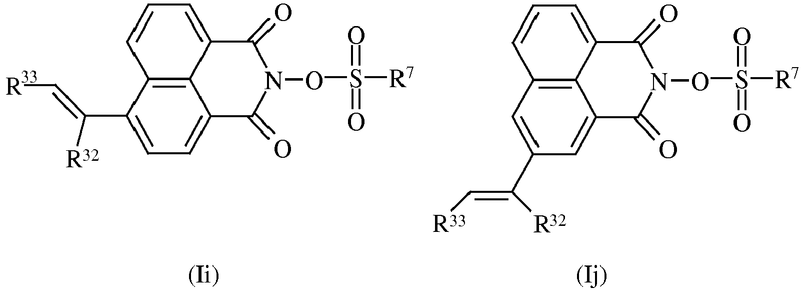 wherein
R³² and R³³ may be the same or different, may be connected to from an alicylic or heterocyclic group and are independently selected from the group consisting of
- a hydrogen atom,
- a cyano group,
- an aliphatic, alicyclic or heterocyclic group having a carbon number of from 1 to 18 in which one or more hydrogen atoms may be substituted by a halogen atom,
- an aliphatic group having a carbon number of from 1 to 18 which comprises at least one moiety selected from the group consisting of -O-, -S-, -C(=O)-, -C(=O)-O-, -C(=O)-S-, -O-C(=O)-O-, -CN, -C(=O)-NH-, -C(=O)-NR^{a}-, and -C(=O)-NR^{a}R^{b}, wherein R^{a} and R^{b} are each independently an aliphatic group with a carbon number of from 1 to 10, which may be the same or different and may be connected to form an alicyclic or heterocyclic group, and wherein the aliphatic group optionally comprises at least one halogen atom,
and
- an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group,
R⁷ is selected from the group consisting of
- an aliphatic, alicyclic or heterocyclic group having a carbon number of from 1 to 18, which may be substituted by one or more halogen atom(s),
- an aliphatic or alicyclic group having a carbon number of from 1 to 18 which comprises at least one moiety selected from the group consisting of -O-, -S-, -C(=O), -C(=O)-O-, -C(=O)-S-, -O-C(=O)-O-, -CN, -C(=O)-NH-, -O-C(=O)-NH-, -C(=O)NR^{a}-, -O-C(=O)-NR^{a}-, and -C(=O)-NR^{a}R^{b}, wherein R^{a} and R^{b} are as defined above, wherein the aliphatic group optionally comprises at least one halogen atom,
and
- an aryl or heteroaryl group having a carbon number of from 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

8. The composition according to anyone of claims 1 to 7, wherein the molar ratio of the sensitizer compound (i) to the acid generating compound (ii) in the composition is in the range from 0.01 : 1 to 100 : 1.

9. The composition according to anyone of claims 1 to 8, wherein the composition further comprises
iii) at least one compound capable of being imparted with an altered solubility in an aqueous solution in the presence of an acid;
iv) at least one organic solvent; and
v) optionally at least one additive being different from components (i), (ii), (iii) and (iv).

10. The composition according to claim 9, wherein the organic solvent (iv) is PGMEA.

11. The composition according to claim 9 or 10, wherein the composition comprises:
(i) 0.01 to 40 wt.-% of the sensitizer compound;
(ii) 0.01 to 10 wt.-% of the acid generating compound;
(iii) 5 to 90 wt.-% of the compound capable of being imparted with an altered solubility in an aqueous solution in the presence of an acid component;
(v) 0 to 10 wt.-% of the further additive;
wherein the reminder in the composition is the organic solvent (iv).

12. A process for producing a composite comprising a substrate and a coating that is applied onto the substrate in a patterned structure, the process comprising the steps of
(α1) applying a layer of the composition according to anyone of claims 9 to 11 onto the surface of the substrate and at least partial removal of the organic solvent (iv);
(α2) exposing selected areas of the layer to electromagnetic radiation, thereby releasing an acid from the acid generating compound (ii) in the areas exposed to the electromagnetic radiation;
(α3) optionally heating the layer to impart compound (iii) in the areas in which the acid has been released with an altered solubility in an aqueous solution;
(α4) optionally at least partial removal of the layer.

13. A composite comprising a substrate and a coating applied on the surface of the substrate in a patterned or non-patterned structure, wherein the coating comprises
- a sensitizer compound (i) as defined in claim 1 to 3
and
- an acid generating compound (ii) as defined in claims 1 and 4 to 7 or the fragment thereof that remains if in general structure (I) the acid group is cleaved.

14. The use of a combination of a sensitizer compound (i) as defined in claim 1 and an acid generating compound (ii) as defined in claims 1 and 4 to 7 for photoinduced polymerization, photoinduced crosslinking, photoinduced degradation, photoinduced deprotection, photoinduced color change, photoinduced transformation of functional groups or any combination of at least two of them.

15. The use of a combination of a sensitizer compound (i) as defined in claim 1 and an acid generating compound (ii) as defined in claims 1 and 4 to 7 in protective coatings, printing plates, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufacturing.
